# EUROPEAN PATENT APPLICATION

(11) **EP 4 699 575 A1**
(43) Date of publication of application: **25.02.2026**
(21) Application number: 25196981.2
(22) Date of filing: 20.08.2025
(51) Int. Cl.: A61F 2/38

(54) **TOTAL STABILIZING KNEE JOINT PROSTHESIS**

(30) Priority: 21.08.2024 US 202463685360 P
(71) Applicant: Howmedica Osteonics Corp., Mahwah, NJ 07430 (US)
(72) Inventor: SERVIDIO, Damon J., TOWACO, 07082 (US); COLLAZO, Carlos E., OLD GREENWICH, 06870 (US)
(74) Representative: Regimbeau

(57) **Abstract**

A knee joint prosthesis (100, 600, 700, 1000) includes a femoral component (102, 602, 702, 1002) and a tibial component (104, 604, 704, 1004, 1104). The femoral component includes a medial condylar articulation surface (107, 607, 707), a lateral condylar articulation surface (109, 609, 709) and a central box (106, 606, 706, 1006) therebetween, the central box being enclosed on a bone-facing side (108) of the femoral component and open on a tibial-facing side of the femoral component. The tibial component including a medial tibial articulation surface (134, 634, 734), a lateral tibial articulation surface (136, 636, 736) and a post (112, 612, 712, 1012, 1112) therebetween, the post being disposed within the central box of the femoral component. A concave curved surface portion (101, 601, 701) of the box travels along a convex curved surface portion (142, 642, 742, 1042, 1142) of the post over a range of motion of the tibial component relative to the femoral component such that an axis of rotation of the box of the femoral component and the post of the tibial component is coincident over the range of motion.

## Description

### BACKGROUND

Constraint utilized for a knee implant in knee replacement and revision surgeries may vary based on an existing condition of a patient anatomy. Knee replacement and revision requires consideration of an extent of bone and soft tissue sacrifice and preservation to determine optimal implant characteristics, with some circumstances requiring more removal of tissue than others. Severe trauma, bone loss, or the need for bone removal, e.g., when a tumor must be removed, may lead to the need for a surgical plan that requires sacrifice of ligaments in the joint. Such cases require more constraint in the knee implant.

Currently, knee replacement implants are offered in multiple constraint levels ranging from cruciate retaining to a hinge-based constraint, with others in between. However, existing options do not provide a high degree of constraint while still preserving a natural interaction between the femoral and tibial components over a range of motion in the knee joint. Additionally, modifying an existing cruciate retaining implant to a more constrained implant or modifying a hinge-based constraint to a less constrained implant is costly and tedious, as a new implant is required.

Accordingly, a need exists to develop a knee implant system that preserves a natural articulation in the knee joint while also providing significant constraint between the femoral and tibial components to address clinical situations where constraint is needed for stability. Additionally, a need exists to develop an adaptable knee implant kit to improve the ease of modification between implants of varying constraint.

### BRIEF SUMMARY

The present disclosure addresses the need for a versatile knee implant by providing an adaptable knee implant kit and a knee joint prosthesis that is more constrained than a total stabilized implant but less constrained than a hinge implant.

In a first aspect, the present disclosure relates to a knee joint prosthesis. In one embodiment, the prosthesis includes a femoral component including a medial condylar articulation surface, a lateral condylar articulation surface and a central box therebetween. The central box is enclosed on a bone-facing side of the femoral component and open on a tibial-facing side of the femoral component. A portion of an internal volume of the central box is defined by a box medial surface, a box lateral surface and a box central surface. The box central surface separates the box medial surface and the box lateral surface. The box medial and lateral surfaces are flat and the box central surface has a concave curved surface portion in an anterior-posterior direction. The prosthesis further includes a tibial component including a medial tibial articulation surface, a lateral tibial articulation surface and a post therebetween. The post includes a post medial surface, a post lateral surface and a post central surface therebetween. The post is disposed within the central box of the femoral component. The post medial surface is complementary to the box medial surface, the post lateral surface is complementary to the box lateral surface, and the post central surface is complementary to the box central surface. The concave curved surface portion of the box central surface travels along a convex curved surface portion of the post central surface over a range of motion of the tibial component relative to the femoral component such that an axis of rotation of the box central surface of the femoral component and the post central surface of the tibial component is coincident over the range of motion.

In some examples, the femoral component and the tibial component are restrained with respect to each other along a medial-lateral direction.

In some examples, at least one of the medial and lateral condylar articulation surfaces and the medial and lateral tibial articulation surfaces rotates about the axis of rotation over the range of motion.

In some examples, the range of motion extends from 0 degrees flexion to 110 degrees flexion.

In some examples, the bone-facing side of the femoral component includes cement pockets for fixation of the femoral component to a bone. In other examples, the bone-facing side of the femoral component includes ingrowth surfaces for biological fixation of the femoral component to a bone. The ingrowth surfaces can be cemented or press-fitted onto the bone.

In some examples, the knee joint prosthesis includes a proximally extending boss having an internally tapered bore for mating with a press-fit stem.

In other examples, the knee joint prosthesis includes a proximally extending boss having an internally threaded bore for mating with a threaded cement stem.

In a second aspect, the present disclosure relates to an implant including a femoral component including a first condylar surface, a second condylar surface, and a box positioned between the first and the second condylar surfaces, the box including a cavity. The implant further includes a tibial component including a first curved surface, a second curved surface, and a post between the first and second curved surfaces. The first and second curved surfaces are complementary to the first and the second condylar surfaces, respectively. A first surface on the post of the tibial component is operatively connected to a second surface within the cavity of the femoral component. When the femoral component articulates relative to the tibial component a medial-lateral movement of the femoral component relative to the tibial component is restrained over a first range of motion, and anterior-posterior movement of the femoral component relative to the tibial component is restrained over a second range of motion.

In some examples, the second surface within the cavity of the femoral component is a concave curved surface portion in an anterior-posterior direction, and the first surface of the post is a convex curved surface portion and wherein the second surface travels along the first surface of over the first range of motion such that an axis of rotation of the second surface of the femoral component and the first surface of the tibial component is coincident over the first and second range of motion.

In some examples, the femoral component articulates relative to the tibial component over the first range of motion and an anterior-posterior movement of the femoral component relative to the tibial component is restrained over the second range of motion and wherein the second range of motion ranges from 0 degrees flexion to 45 degrees flexion.

In some examples, wherein the first surface on the post of the tibial component includes a protrusion that is operatively connected to the second surface within the cavity of the femoral component over the second range of motion.

In some examples, the first surface defines a partially cylindrical end portion of the post.

In some examples, the first range of motion and the second range of motion range from 0 degrees flexion to 110 degrees flexion. Over the first range of motion and the second range of motion range, the second surface is superior to the first surface.

In some examples, the convex curved surface portion of the post central surface is only curved in an anterior-posterior direction. In other examples, the convex curved surface portion of the post central surface is curved in an anterior-posterior and in a medial-lateral direction.

In some examples, a length of the post is uninterrupted by the femoral component.

In some examples, the femoral component includes a bone-facing side having cement pockets for fixation of the femoral component to a bone. In other examples, the femoral component includes a bone-facing side having ingrowth surfaces for biological fixation of the femoral component to a bone. The ingrowth surfaces can be cemented or press-fitted onto the bone.

In some examples, the implant includes a proximally extending boss having an internally tapered bore for mating with a press-fit stem. In other examples, the implant includes a proximally extending boss having an internally threaded bore for mating with a threaded cement stem.

In some examples, the post of the tibial component includes a post medial surface and a post lateral surface and the cavity of the femoral component includes a medial surface and a lateral surface. The post medial and lateral surfaces may be angled with respect to the medial and lateral surfaces of the cavity at an angle of less than 60 degrees.

In a third aspect, the present disclosure relates to a kit including a femoral insert, a first tibial insert, and a second tibial insert. The femoral insert is configured to be received on a bone-facing side of a femoral articular component including first and second articulation surfaces. The femoral insert includes a box with a cavity between medial and lateral condylar portions of the femoral insert. The first tibial insert is configured to be received on a joint-facing side of a tibial baseplate. The first tibial insert includes a peg extending away from the tibial baseplate when the first tibial insert is attached to the tibial baseplate. The second tibial insert includes a medial articulation surface, a lateral articulation surface, and a post therebetween. A cavity of the post is configured to receive the peg, and the post is receivable within the cavity of the box of the femoral insert. When the femoral insert is received in the femoral articular component to define a femoral implant and the first and second tibial inserts are received on the tibial baseplate to define a tibial implant, the femoral implant rotates relative to the tibial implant such that an axis of rotation of an inner surface of the box of the femoral implant and an end surface of the post of the tibial implant is coincident over a range of motion.

In some examples, the kit further includes the femoral articular component and the tibial baseplate. The femoral articular component is configured to receive the femoral insert and the tibial baseplate is configured to receive the first tibial insert and the second tibial insert. The femoral articular component includes a medial condylar articulation surface having a medial peg, a lateral condylar articulation surface having a lateral peg, and a central opening therebetween.

In some examples, the box of the femoral insert is enclosed on a bone-facing side of the femoral articular component and open on a tibial-facing side of the femoral articular component. A portion of an internal volume of the box is defined by the inner surface of the box. The inner surface of the box is complimentary to the end surface of the post.

In some examples, the end surface of the post is a convex curved surface and the inner surface of the box is a concave curved surface, the post of the second tibial insert being disposed within the box of the femoral insert.

In some examples, the medial and the lateral condylar portions of the femoral insert include a medial opening and a lateral opening complementary to the medial and lateral pegs of the femoral articular component.

In some examples, the first tibial insert includes a platform at a base of the peg and surrounding the base of the peg, the platform being releasably engageable with the tibial baseplate.

In other examples, the first tibial insert includes an elongated peg, the peg having an end including a bore. The elongated peg is configured to be inserted into a boss extending from the tibial baseplate so that the end protrudes from the tibial baseplate after the elongated peg is fully inserted into the boss.

In some examples, the kit further includes a bone cement configured to secure the tibial baseplate and the tibial insert together.

In some examples, the kit further includes a stem configured to be engaged with an adapter stem on the femoral insert.

In some examples, the femoral insert further includes a fastener configured to secure the stem to the femoral insert.

In some examples, the bone-facing side of the femoral insert includes an ingrowth surface to secure the femoral articular component directly to a bone. In some examples, the kit further includes a bone cement configured to secure the femoral insert to the femoral articular component.

In some examples, the femoral insert, first tibial insert, and second tibial insert include a polymer, a reinforced polymer composite, or a biocompatible metal.

In a fourth aspect, the present disclosure relates to a method of implanting a knee joint prosthesis. The method includes attaching a femoral component to a femur; attaching a tibial component to a tibia; and rotationally coupling the femoral component to the tibial component. The femoral component includes a central box having an internal volume defined by a box medial surface, a box lateral surface and a box central surface. The tibial component includes a post defined by a post medial surface, a post lateral surface and a post central surface therebetween, the post being disposed within the central box of the femoral component. An axis of rotation of the box central surface and the post central surface is coincident over a range of motion.

In some examples, attaching the femoral component to a femur includes attaching a stem to a femoral component.

In some examples the femoral component includes a medial condylar articulation surface, a lateral condylar articulation surface and the central box therebetween, the central box is enclosed on a bone-facing side of the femoral component and open on a tibial-facing side of the femoral component, the box central surface separating the box medial surface and the box lateral surface.

In some examples, the tibial component includes a medial tibial articulation surface, a lateral tibial articulation surface and the post therebetween.

In some examples, the post medial surface is complementary to the box medial surface, the post lateral surface is complementary to the box lateral surface, and the post central surface is complementary to the box central surface.

In some examples, rotationally coupling the femoral component to the tibial component includes inserting the post of the tibial component into the box of the femoral component.

In some examples, a concave curved surface portion of the box central surface travels along a convex curved surface portion of the post central surface over a range of motion of the tibial component relative to the femoral component.

In some examples, attaching the stem to the femoral component includes applying a bone cement on an interface between the stem and the femoral component. In other examples, attaching the stem to the femoral component includes press-fitting the stem into the femoral component.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIG. 1 is a side cross-sectional view of a knee joint prosthesis in accordance with an embodiment of the present disclosure.
FIG. 2 is a rear cross-sectional view of the knee joint prosthesis of FIG. 1.
FIG. 3 is a perspective view of a femoral component of the knee joint prosthesis of FIG. 1.
FIG. 4 is a perspective view of the femoral component of the knee joint prosthesis of FIG. 1.
FIG. 5 is a perspective view of the tibial component of the knee joint prosthesis of FIG. 1.
FIG. 6 is a side view of the tibial component of the knee joint prosthesis of FIG. 1.
FIG. 7 is a rear view of a tibial component of the knee joint prosthesis of FIG. 1.
FIG. 8 is a side cross-sectional view of a knee joint prosthesis in accordance with an embodiment of the present disclosure.
FIG. 9 is a rear cross-sectional view of the knee joint prosthesis of FIG. 8.
FIG. 10 is a perspective view of a femoral component of the knee joint prosthesis of FIG. 8.
FIG. 11 is a perspective view of the tibial component of the knee joint prosthesis of FIG. 8.
FIG. 12 is a side view of the tibial component of the knee joint prosthesis of FIG. 8.
FIG. 13 is a rear view of a tibial component of the knee joint prosthesis of FIG. 8.
FIG. 14 is a side cross-sectional view of a knee joint prosthesis in accordance with an embodiment of the present disclosure.
FIG. 15 is a rear cross-sectional view of the knee joint prosthesis of FIG. 14.
FIG. 16 is a perspective view of a femoral component of the knee joint prosthesis of FIG. 14.
FIG. 17 is a perspective view of the tibial component of the knee joint prosthesis of FIG. 14.
FIG. 18 is a side cross-sectional view of the tibial component of the knee joint prosthesis of FIG. 14.
FIG. 19 is a rear cross-sectional view of a tibial component of the knee joint prosthesis of FIG. 14.
FIG. 20 is a side cross-sectional view of the knee joint prosthesis of FIG. 1 at negative 15 degrees hyperextension.
FIG. 21 is a side cross-sectional view of the knee joint prosthesis of FIG. 1 at 0 degrees extension.
FIG. 22 is a side cross-sectional view of the knee joint prosthesis of FIG. 1 at 75 degrees flexion.
FIG. 23 is a side cross-sectional view of the knee joint prosthesis of FIG. 1 at 110 degrees flexion.
FIG. 24 is an exploded perspective view of a knee joint prosthesis in accordance with another embodiment of the present disclosure.
FIG. 25 is an exploded perspective view of the knee joint prosthesis of FIG. 24.
FIG. 26 is an exploded perspective view of a femoral component of the knee joint prosthesis of FIG. 24.
FIG. 27 is an exploded perspective view of the femoral component of the knee joint prosthesis of FIG. 24.
FIG. 28 is an exploded perspective view of the femoral component of the knee joint prosthesis of FIG. 24 having a stem in accordance with another embodiment of the present disclosure.
FIG. 29 is a perspective view of a femoral insert in accordance with another embodiment of the present disclosure.
FIG. 30 is a perspective view of a femoral insert in accordance with another embodiment of the present disclosure.
FIG. 31 is an exploded perspective view of a knee joint prosthesis in accordance with another embodiment of the present disclosure.
FIG. 32 is an exploded perspective view of a knee joint prosthesis in accordance with another embodiment of the present disclosure.
FIG. 33 is an exploded perspective view of a tibial component of a knee joint prosthesis in accordance with another embodiment of the present disclosure.
FIG. 34 is an exploded perspective view of a tibial component of the knee joint prosthesis of FIG. 33.
FIG. 35 is an exploded perspective view of the tibial component of the knee joint prosthesis of FIG. 33.
FIG. 36 is an exploded perspective view of the tibial component of the knee joint prosthesis of FIG. 33.
FIG. 37 is a side view of the tibial component of the knee joint prosthesis of FIG. 33.
FIG. 38 is a side view of the tibial component of the knee joint prosthesis of FIG. 33.
FIG. 39 is a flowchart illustrating a method of implanting the knee joint prosthesis of FIG. 1.
FIG. 40 is a flowchart illustrating a method of assembling the knee joint prosthesis of FIG. 24 and FIG. 33.
FIG. 41 is a side cross-sectional view of one step in a method of use of a knee joint prosthesis in accordance with an embodiment of the present disclosure.
FIG. 42 is a side cross-sectional view of one step in the method of use of the knee joint prosthesis of FIG. 41.
FIG. 43 is a side cross-sectional view of one step in the method of use of the knee joint prosthesis of FIG. 41.
FIG. 44 is a side cross-sectional view of one step in the method of use of the knee joint prosthesis of FIG. 41.
FIG. 45 are overlapping side cross-sectional views of steps in the method of use of the knee joint prosthesis of FIG. 41.
FIG. 46 is a side view of a tibial component of the knee joint prosthesis of FIG. 41.
FIG. 47 is a side view of a tibial component in accordance with an embodiment of the present disclosure.
FIG. 48 is a perspective view of a tibial component in accordance with an embodiment of the present disclosure.
FIG. 49 is a side view of the tibial component of FIG. 48.
FIG. 50A is a side view of the tibial component of FIG. 48.
FIG. 50B is a cross-sectional top view of the tibial component of FIG. 50A along line A-A. FIG. 50C is a cross-sectional top view of the tibial component of FIG. 50A along line B-B.
FIG. 51 is a top view of the tibial component of FIG. 48.
FIG. 52 is a top view of the tibial component of FIG. 48.
FIG. 53 is a perspective view of a femoral component in accordance with an embodiment of the present disclosure.
FIG. 54 is a front view of the femoral component of FIG. 53.
FIG. 55 is an exploded perspective view of a tibial component of a knee joint prosthesis in accordance with another embodiment of the present disclosure.
FIG. 56 is an exploded perspective view of the tibial component of FIG. 55.
FIG. 57 is a cross-sectional side view of the tibial component of FIG. 55 and a femoral component in accordance with another embodiment of the present disclosure.
FIG. 58 is a side view of the tibial component of FIG. 55.
FIG. 59 is a perspective view of a second tibial insert in accordance with another embodiment of the present disclosure.
FIG. 60 is a side view of the femoral component of FIG. 41.

### DETAILED DESCRIPTION

In describing preferred embodiments of the disclosure, reference will be made to directional nomenclature used in describing the human body. It is noted that this nomenclature is used only for convenience and that it is not intended to be limiting with respect to the scope of the present disclosure. As used herein unless stated otherwise, the term "proximal" means closer to the heart and the term "distal" means further from the heart. The term "anterior" means toward the front part of the body, and the term "posterior" means toward the back part of the body. The term "medial" means closer to or toward the midline of the body, and the term "lateral" means further from or away from the midline of the body. The term "inferior" means closer to or toward the feet, and the term "superior" means closer to or toward the crown of the head. In addition, the terms "about," "generally," and "substantially" are intended to mean that slight deviations from absolute are included within the scope of the term so modified.

In one aspect, the present disclosure relates to a knee joint prosthesis providing a constrained total stabilizing knee joint. Examples of such a knee joint prosthesis are shown in the figures of the present application. In some examples, the knee joint prosthesis may include a femoral component and a tibial component. In other examples, the knee joint prosthesis may include one or more femoral components and one or more tibial components.

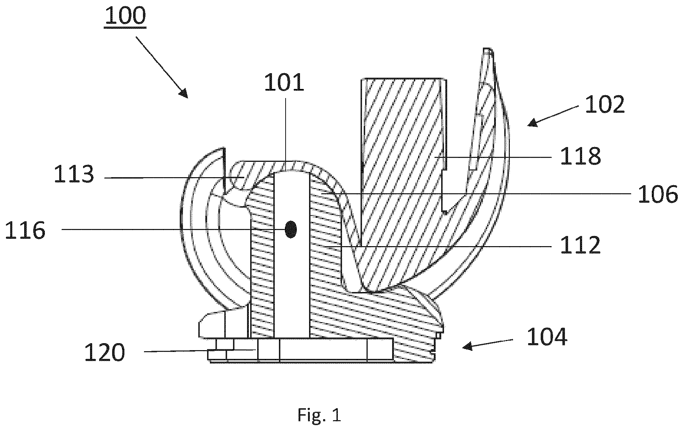

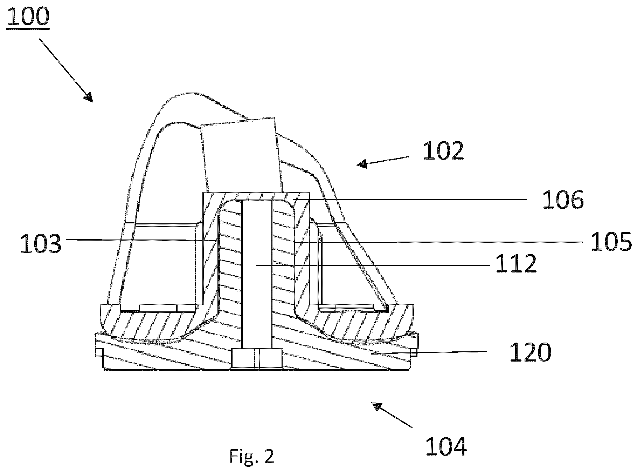

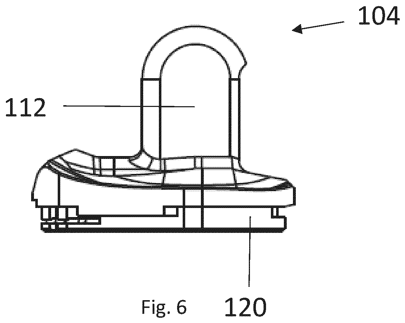

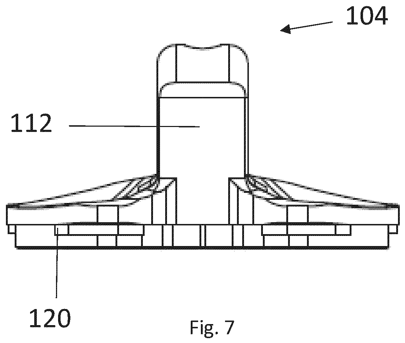

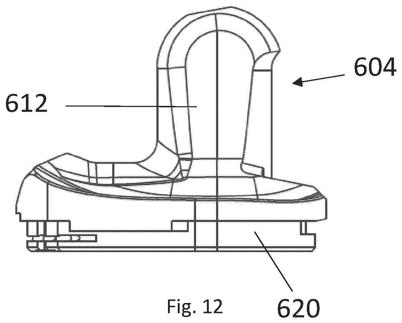

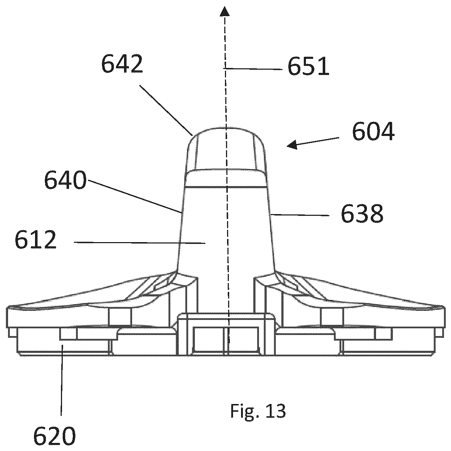

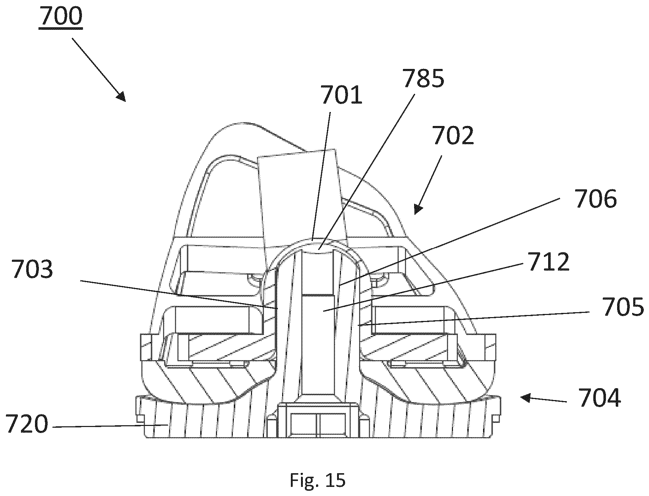

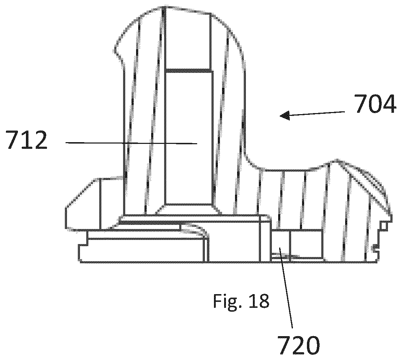

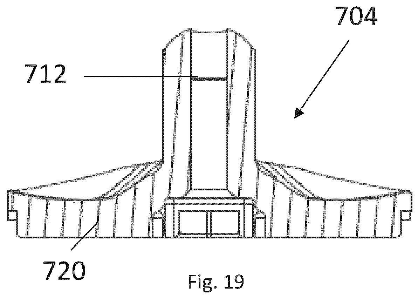

In general, knee joint prosthesis 100 may include a femoral component 102 and a tibial component 104. As shown in FIGS. 1 and 2, the interaction between the components includes operative engagement between a central box 106 of femoral component 102 with a post 112 protruding from a baseplate 120 of tibial component 104. Although FIGS. 1-19 are described in reference to a left-side knee prosthesis implant, it should be appreciated that the principles and features of the described embodiment may be applied in equal measure to a right-side knee prosthesis implant although not shown in the figures.

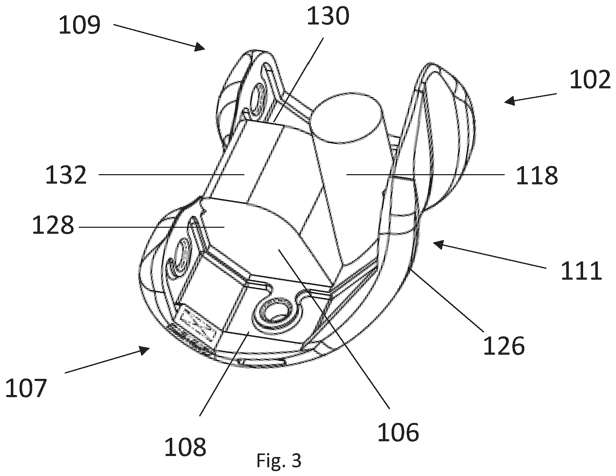

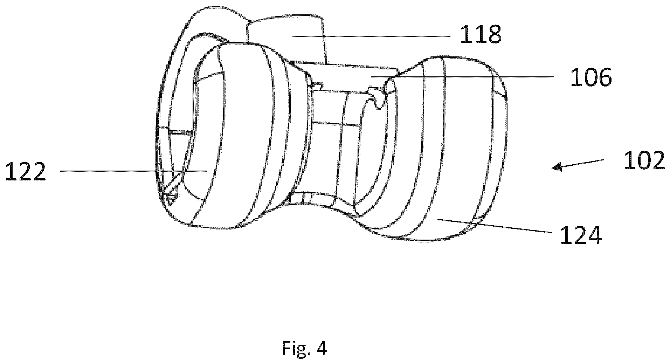

As shown in FIG. 3, femoral component 102 includes a medial condylar portion 107, a lateral condylar portion 109, and an anterior portion 111. As shown in FIGS. 3 and 4, femoral component 102 includes a medial condylar articulation surface 124 and a lateral condylar articulation surface 122 on medial condylar portion 107 and lateral condylar portion 109, respectively. Central box 106 is positioned between lateral and medial articulation surfaces 122, 124 separating medial condylar portion 107 from lateral condylar portion 109. Central box 106 is enclosed on a bone-facing surface 108 of femoral component 102 that engages an end of a femur bone. Central box 106 is open on a tibial facing side 126 of femoral component 102 that engages tibial component 104. Central box 106 includes an external box medial surface 128, an external box lateral surface 130, and an external box central surface 132. In some examples, as shown in FIG. 3, external box central surface 132 may include one or more planar surfaces extending in an anterior-posterior direction.

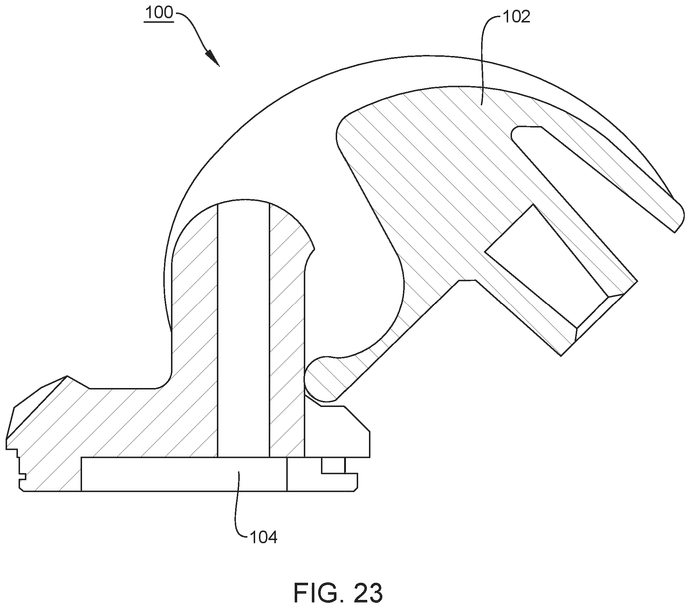

Referring back to FIGS. 1 and 2, central box 106 includes an internal volume that is defined by a box medial surface 105, a box lateral surface 103, and a box central surface 101. Box central surface 101 separates box medial surface 105 and box lateral surface 103. Box medial surface 105 and box lateral surface 103 are flat, while box central surface 101 has a concave curved surface portion. The concave curved surface portion generally extends in an anterior-posterior direction. In some examples, and as shown in FIG. 1, box central surface 101 includes a protruding portion 113 extending from a posterior end of the concave curved surface portion. Protruding portion 113 may function as a cam such that in extreme flexion, such as that shown in FIG. 23, protruding portion 113 engages with a posterior surface of a post 112 of the tibial component 104, facilitating roll-back of the femur with respect to the tibia.

Anterior portion 111 of femoral component 102 extends from an end each of medial condylar portion 107, lateral condylar portion 109, and central box 106. Femoral component 102 further includes a boss 118 extending proximally from anterior portion 111 at a location adjacent to central box 106. As depicted, central box 106 is configured to restrain a post of a tibial component so that the components of the joint only include one degree of freedom over a large range of flexion, described in greater detail elsewhere in the present disclosure. In alternative examples, central box 106 may provide varying levels of varus and valgus constraint as well as various levels of translational constraint in the anterior posterior direction.

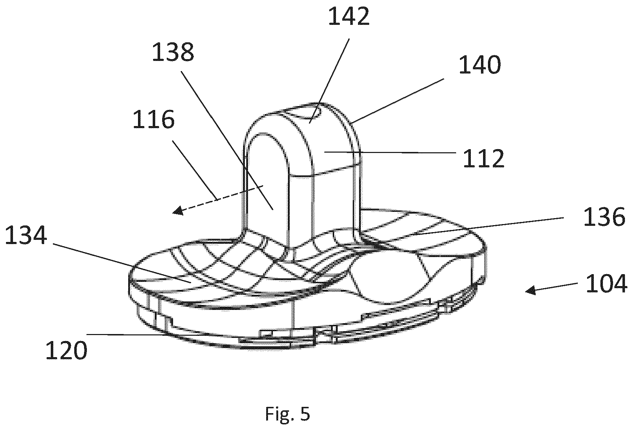

As shown in FIGS. 5-7, tibial component 104 includes a medial tibial articulation surface 134 and a lateral tibial articulation surface 136. Tibial component 104 further includes a post 112 positioned between medial and lateral tibial articulation surfaces 134, 136. The post 112 includes a post medial surface 138, a post lateral surface 140, and a post central surface 142 between medial and lateral surfaces of the post. Post medial surface 138 and post lateral surface 140 include a flat surface extending to the medial and lateral tibial articulation surfaces 134, 136, while post central surface 142 has a convex curved surface portion. In some examples, post central surface 142 may be hemicylindrical. When femoral component 102 engages tibial component 104, post 112 is disposed within central box 106 of femoral component, and medial tibial articulation surface 134 and lateral tibial articulation surface 136 engage medial condylar articulation surface 124 and lateral condylar articulation surface 122, respectively.

Referring back to FIGS. 1 and 2, the flat surface of post medial surface 138 is complementary to the flat surface of box medial surface 105 and the flat surface of post lateral surface 140 is complementary to the flat surface of box lateral surface 103. Additionally, the convex curved surface of post central surface 142 is complementary to the concave curved surface of box central surface 101. In the depicted example, post 112 of tibial component 104 engages central box 106 of femoral component 102 such that femoral component 102 and tibial component 104 are restricted to movement only about a single axis of rotation 116 over a predetermined range of motion. As shown in FIG. 2, central box 106 of femoral component 102 and post 112 of tibial component 104 provide valgus-varus constraint. For example, this restriction of movement may be present over a range of rotational positions from 0 degrees of extension to 110 degrees of flexion. In other examples, the box and the post may be modified to adjust this range.

Reference will now be made to additional examples of the knee joint prosthesis of the present disclosure. Wherever possible, the same or like reference numbers will be used throughout the drawings to refer to the same or like features of prosthesis 100 but within a different 100-series of numbers, e.g., the 600-series or 700-series of numbers. The differences between different knee joint prosthesis will be focused on below, with the understanding that other portions of the knee joint prosthesis may be similar or even identical.

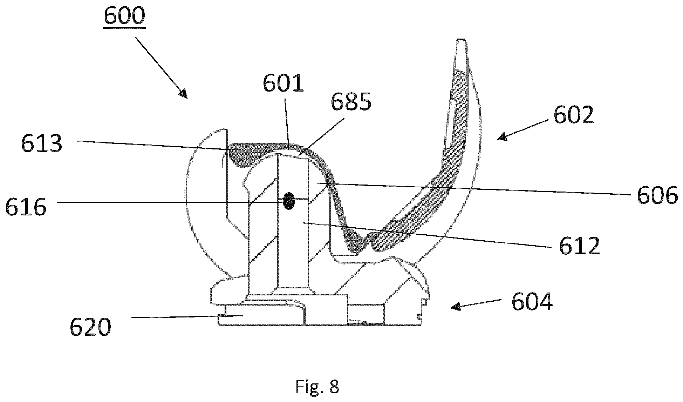

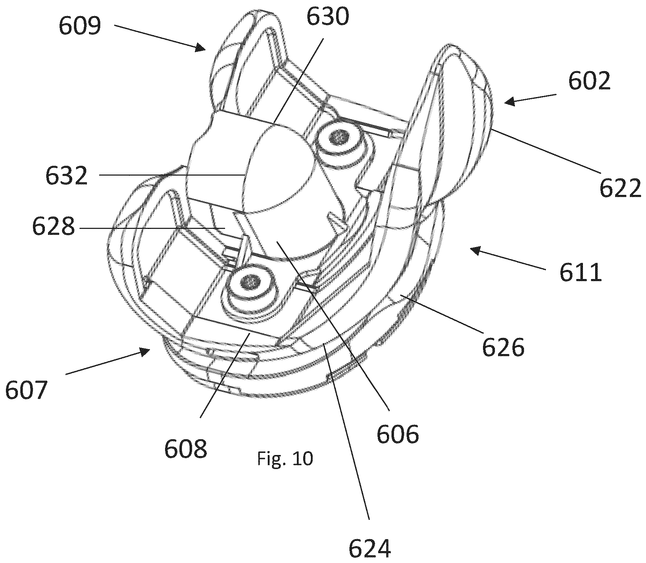

Beginning with FIGS. 8-9, a knee joint prosthesis 600 is shown, the design of which is similar to that of knee joint prosthesis 100. However, knee joint prosthesis 600 includes a femoral component 602 having a central box 606 as shown in FIG. 10. Central box 606 includes an external box medial surface 628, an external box lateral surface 630, and an external box central surface 632. As shown in FIG. 10, external box central surface 632 is a curved surface. Referring back to FIGS. 8-9, central box 606 includes an internal volume that is defined by a box medial surface 605, a box lateral surface 603, and a box central surface 601. Box central surface 601 separates box medial surface 605 and box lateral surface 603. Box medial surface 605 and box lateral surface 603 are flat, while box central surface 601 has a spherical or rounded shape, which provides increased anterior posterior stability during flexion relative to a hemicylindrical shape.

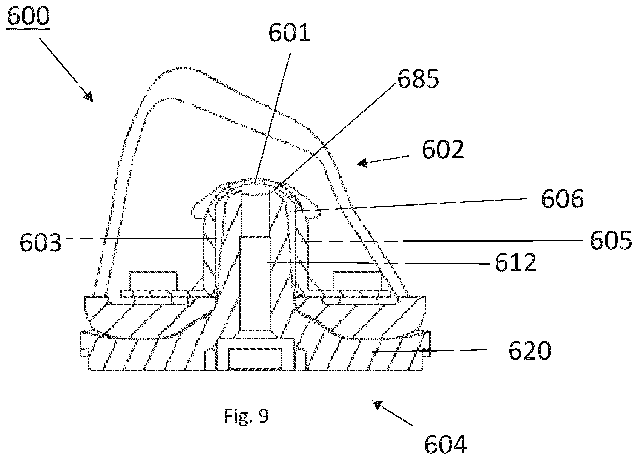

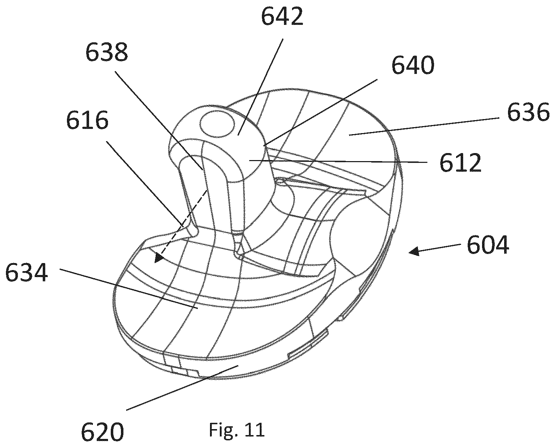

As shown in FIGS. 11-13, tibial component 604 includes a post 612 having a post medial surface 638, a post lateral surface 640, and a post central surface 642 between medial and lateral surfaces of the post. Post medial surface 638 and post lateral surface 640 include respective surfaces extending from post central surface 642 to the medial and lateral tibial articulation surfaces 634, 636, while post central surface 642 has a spherical or round surface portion. Referring back to FIGS. 8-9, the radius of curvature of the post central surface 642 is complimentary to the radius of curvature of the box central surface 601. Post central surface 642 is separated from box central surface 601 by a gap 685. Gap 685 facilitates articulation of the femoral component relative to the tibial component. As shown in FIG. 9, central box 606 of femoral component 602 and post 612 of tibial component 604 are sized to allow for some valgus-varus movement. In some examples, post medial and lateral surface 638, 640 extend from medial and lateral tibial articulation surfaces 634, 636 at an angle of less than 60 degrees with respect to a central longitudinal axis 651 of post 612. In some examples, the angle may be an angle in a range between 1 and 60 degrees. In some examples, medial and lateral tibial articulation surfaces 634, 636 may extend at an angle ranging from 25 to 30 degrees with respect to central longitudinal axis 651. In other examples, medial and lateral tibial articulation surfaces 634, 636 may extend at an angle 5, 10, 15, 20 or 30 degrees with respect to central longitudinal axis 651. Box medial and lateral surfaces 605, 603 are substantially perpendicular to a medial-lateral axis when femoral component 602 is attached to the femur.

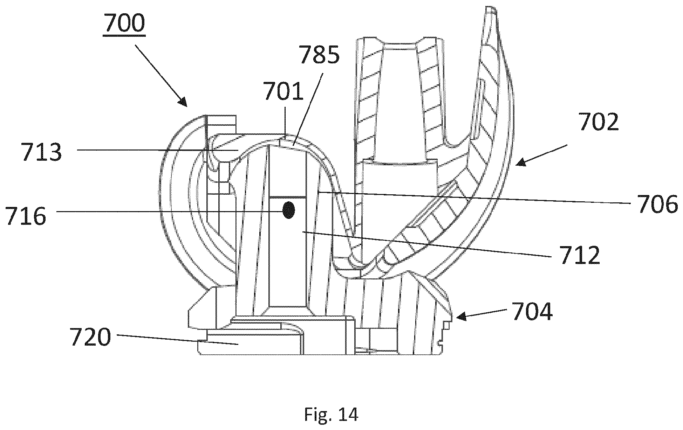

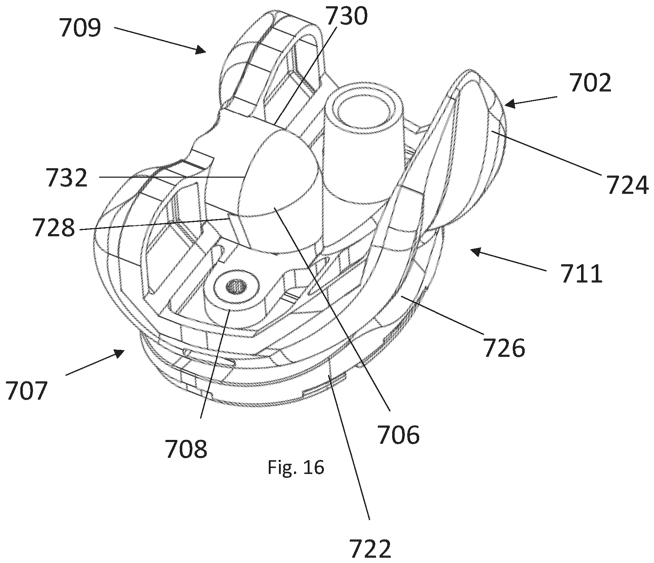

A further knee joint prosthesis 700 is shown in FIGS. 14-15, the design of which is similar to that of knee joint prosthesis 100. However, as shown in FIG. 16, knee joint prosthesis 700 includes a central box 706 having an external box medial surface 728, an external box lateral surface 730, and an external box central surface 732. As shown in Fig. 16, external box central surface 732 is a surface that is curved in multiple directions. Referring back to FIGS. 14-15, central box 706 includes an internal volume that is defined by a box medial surface 705, a box lateral surface 703, and a box central surface 701. Box central surface 701 separates box medial surface 705 and box lateral surface 703. Box medial surface 705 and box lateral surface 703 are flat, while box central surface 701 has a spherical or rounded shape, similar to that of box central surface 601.

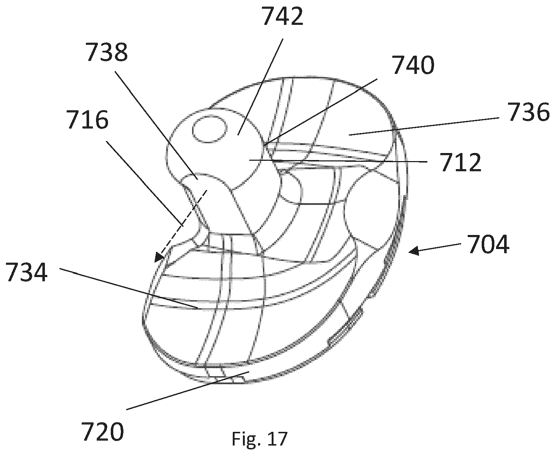

As shown in FIGS. 17-19, tibial component 704 includes a post 712 having a medial surface 738, a post lateral surface 740, and a post central surface 742 between medial and lateral surfaces of post. Post medial surface 738 and post lateral surface 740 include a flat surface extending to medial and lateral tibial articulation surfaces 734, 736, while post central surface 742 has a spherical or round surface portion. Referring back to FIGS. 14-15, the radius of curvature of the post central surface 742 is complimentary to the radius of curvature of the box central surface 701. Post central surface 742 is separated from box central surface 701 by a gap 785. Gap 785 facilitates articulation of the femoral component relative to the tibial component. The flat surface of post medial surface 738 is complementary to the flat surface of box medial surface 705 and the flat surface of post lateral surface 740 is complementary to the flat surface of box lateral surface 703. Thus, in some examples, post medial surface 738 may be parallel to box medial surface 705 and post lateral surface 740 may be parallel to box lateral surface 703.

An overall range of motion of the joint implant may be greater than the range of motion exhibiting a single axis of rotation. Thus, in some examples, the range of rotational positions of the joint implant may range from 15 degrees of hyperextension to 110 degrees of flexion, where the range of motion with a single axis of rotation is from 0 to 110 degrees of flexion within the larger range. In some examples, the single axis may be an epicondylar axis. Over the range of motion, the concave curved surface portion of box central surface 101 travels along a convex curved surface portion of post central surface 142 such that axis of rotation 116 of box central surface 101 of the femoral component and post central surface 142 of tibial component 104 is coincident. Put another way, femoral component 102 rotates with respect to tibial component 104 about axis of rotation 116 with one degree of freedom, thereby providing a total stabilizing constraint knee implant.

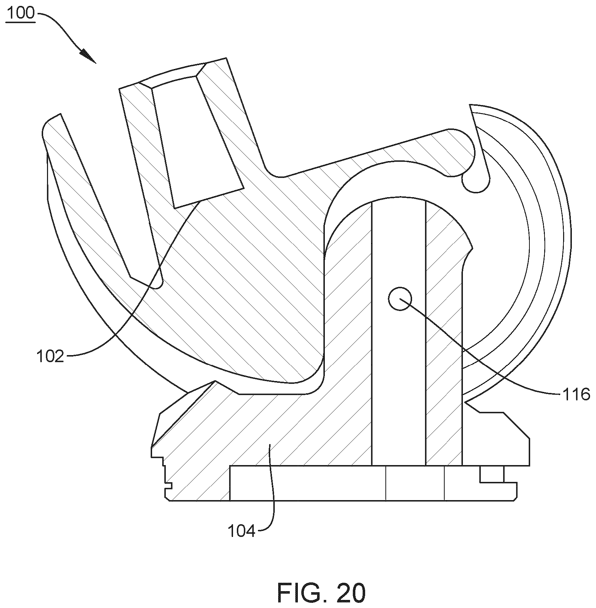

Further detail regarding the functional relationship between the femoral and tibial components is shown in FIGS. 20-23 for knee joint prosthesis 100. Femoral component 102 and tibial component 104 rotatable relative to each other about axis of rotation 116 or the epicondylar axis. In some examples, axis of rotation 116 may extend through the post medial and lateral surfaces 138, 140 such that axis of rotation 116 is perpendicular to surfaces 138, 140. Axis of rotation 116 is located at a center point of a circumference coincident with box central surface 101 and post central surface 142. Femoral component 102 and tibial component 104 are restrained with respect to each other along a medial-lateral direction. Medial condylar articulation surface 124 and lateral condylar articulation surface 122 both rotate about axis of rotation 116 over the range of motion. Additionally, such axis of rotation 116 may, at the same time, be an axis of rotation for the respective articular surfaces 122, 124, 134, 136 of the femoral and tibial components over the range of motion. As mentioned above, a range of motion with a common axis of rotation may be between 0 degrees extension and 110 degrees flexion.

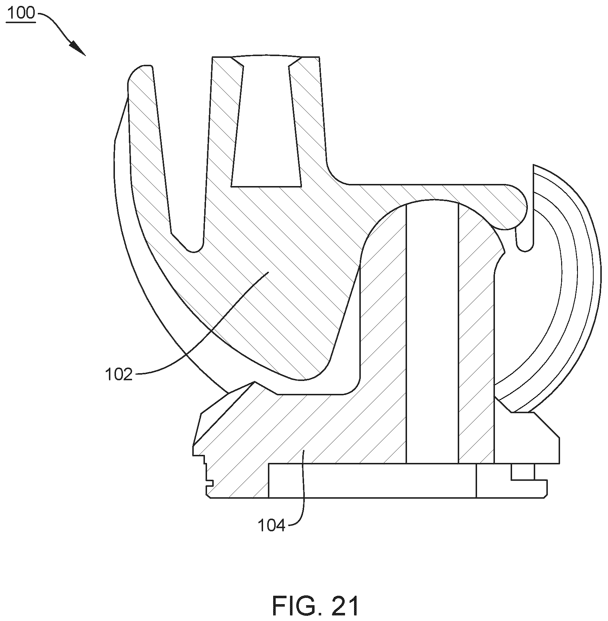

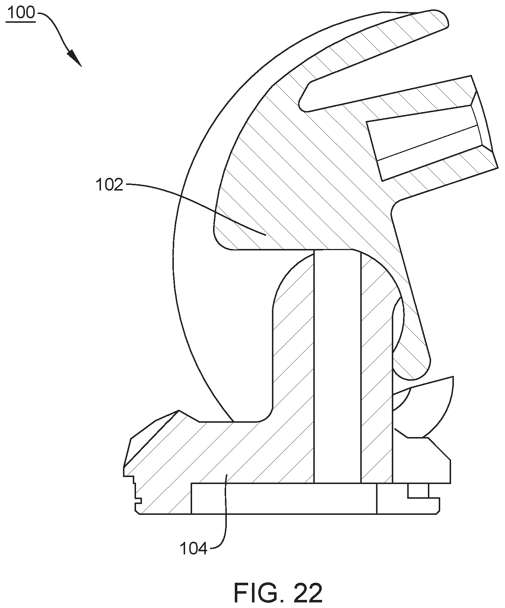

FIGS. 20-23, illustrate rotational positions encompassing such range along with a rotational position outside of the congruent range of motion at 15 degrees hyperextension (-15 degrees) in FIG. 20. At a position outside of the congruent range of motion in the depicted embodiment, specifically, at 15 degrees hyperextension, the femoral and tibial components move relative to each other such that the axis of rotation of the femoral and tibial components is no longer congruent. In some examples, a range of motion where the femur and tibia remain congruent may be larger than that shown in the depicted embodiment. For example, the implant components may be configured so that the range where the femur and tibia are congruent may extend from 15 degrees hyperextension to 110 degrees flexion. In other examples, the range of congruency may only extend from 0 to 110 degrees flexion, as shown in FIGS. 20-23. Of the rotational positions within the constrained range of motion for the depicted embodiment, FIG. 21 illustrates femoral component 102 at a 0 degree flexion relative to the tibial component 104. FIG. 22 illustrates femoral component 102 at a 75 degree flexion relative to tibial component 104. FIG. 23 illustrates femoral component 102 at a 110 degree flexion relative to tibial component 104. Knee joint prostheses 600, 700 may have similar performance characteristics, e.g., range of motion with a single rotational axis, as described above for knee joint prosthesis 100. Notwithstanding the above, it should be appreciated that flexion of a knee joint prosthesis, such as prosthesis 100, 600 or 700, is not limited to that illustrated in the figures. For example, in some embodiments, an overall range of motion in the joint may be greater than or less than -15 degrees to 110 degrees.

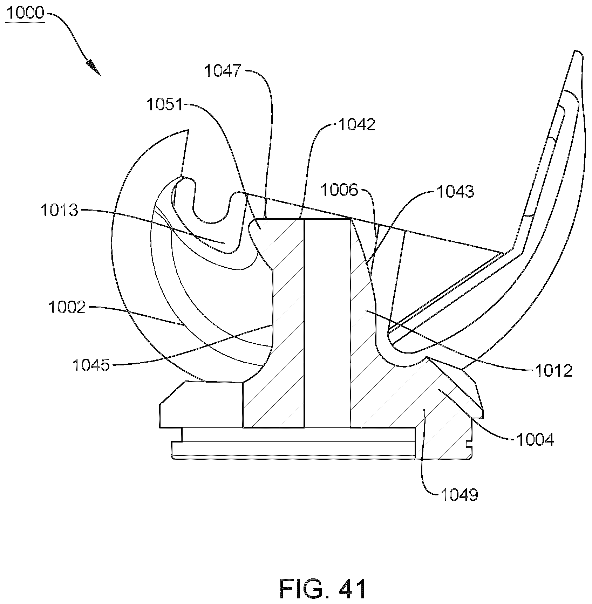

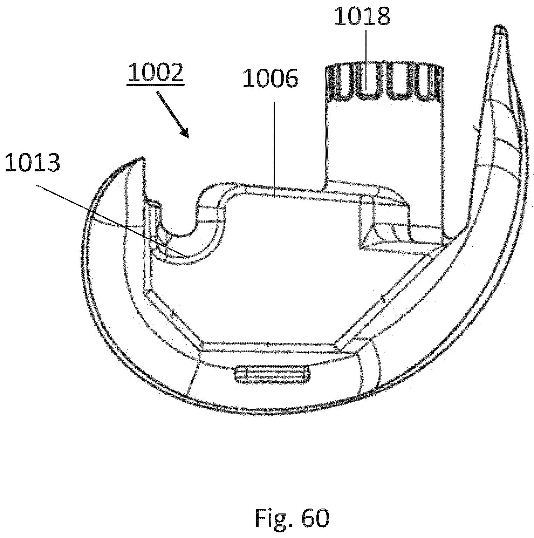

A further knee joint prosthesis 1000 is shown in FIGS. 41-45. For knee joint prosthesis 1000, like reference numerals refer to like elements of knee joint prosthesis 100, unless otherwise noted. As shown in FIGS. 41-45, knee joint prosthesis 1000 includes a femoral component 1002 and a tibial component 1004. The design of femoral component 1002 is similar to that of femoral component 102. In some examples, femoral component 1002 may include a femoral cam 1013 on an inner surface of a central box 1006. Femoral cam 1013 may be a bump or a rounded protrusion on the inner surface of femoral component 102. In some examples, and as shown in FIG. 60, femoral cam 1013 may be a bump or ridge (not shown) on an upper inner surface of central box 1006, proximate a posterior side of central box 1006. Femoral cam 1013 may extend across a width, i.e., in a medial-lateral direction, of the central box 1006. Femoral cam 1013 is configured to articulate with a post of a tibial component.

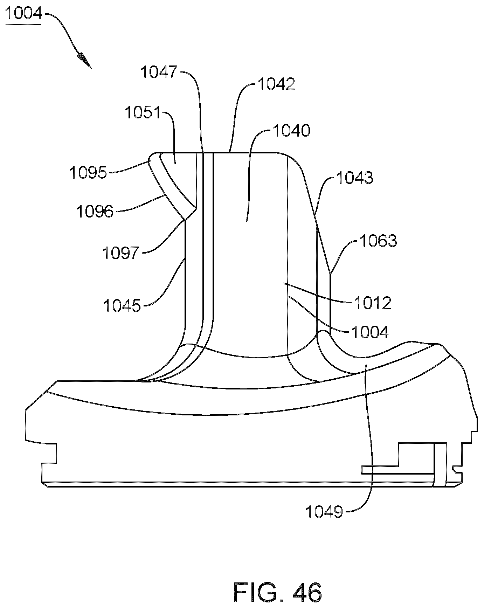

The tibial component 1004 is similar in design to tibial component 104, unless otherwise noted. As shown in FIGS. 41-46, tibial component 1004 includes a post 1012 positioned between medial and lateral tibial articulation surfaces (not shown). Post 1012 includes a post medial surface (not shown), a post lateral surface 1040, and a post central surface 1042 between medial and lateral surfaces of the post. As shown in FIG. 46, post central surface 1042 may include a series of individual surfaces including an anterior surface 1043, a posterior surface 1045, and an upper surface 1047 in between posterior and anterior surfaces. Anterior surface 1043 of post 1012 may be a flat surface extending to a central surface 1049 between the medial and lateral tibial articulation surfaces. In some examples, anterior surface 1043 may include an angled portion extending from upper surface 1047 to a first point 1063 along anterior surface 1043. The angled portion is configured for patella relief during deep flexion of knee joint prosthesis 1000. A flat portion of anterior surface 1043 of post 1012 may extend from the first point to central surface 1049.

As shown in FIG. 46, posterior surface 1045 may be on a side of post 1012 opposite from anterior surface 1043. In some examples, posterior surface 1045 may extend to central surface 1049. posterior surface 1045 may be a flat surface including a protrusion 1051 on an upper end of posterior surface 1045 adjacent upper surface 1047 of post 1012. In some examples, an upper edge of protrusion 1051 of posterior surface 1045 may extend from upper surface 1047 of post 1012. In some examples, upper surface 1047 of post 1012 may be a flat surface. In some examples, as shown in FIG. 46, protrusion 1051 may have a pyramidical shape or sharp edge. In some examples, protrusion 1051 may extend outward from posterior surface 1045 by an amount in a range of 2 to 10 mm. In some examples, protrusion 1051 may extend outward 4 mm from posterior surface 1045. In some examples, an outer surface of protrusion 1051 may include a first curve 1095, a second curve 1096, and a third curve 1097. First curve 1095 may have a radius in a range from 1 to 4 mm. Second curve 1096 may have a radius in a range from 3 to 20 mm. Third curve 1097 may have a radius in a range from 1 to 3 mm.

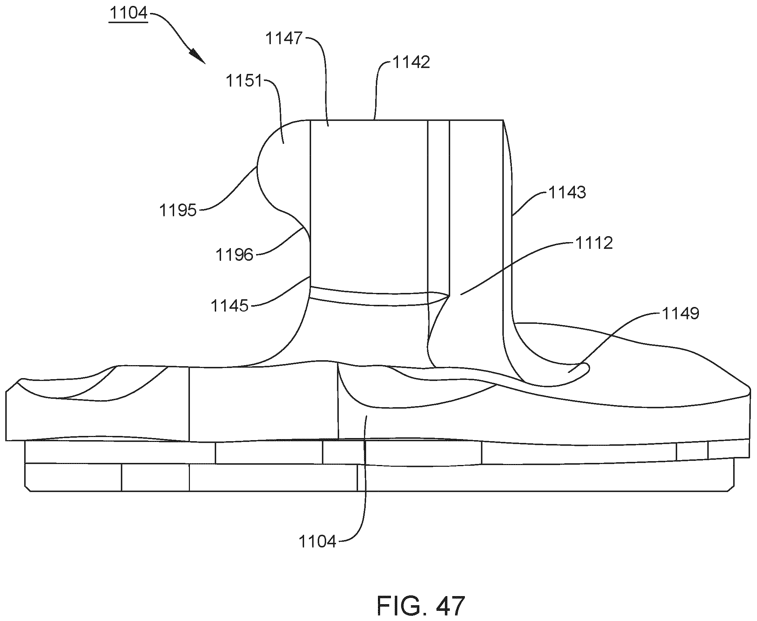

In other examples, and as shown in FIG. 47, a tibial component 1104 may be similar in design to tibial component 1004, however, protrusion 1151 of post 1112 may include a rounded surface or spherical bump. Further, posterior surface 1145 may include a concave portion that extends into a convex portion or protrusion 1151. In some alternative examples, posterior surface 1145 may include a flat portion or straight surface that extends into convex portion or protrusion 1151. In some examples, an outer surface of protrusion 1151 may include a first curve 1195 and a second curve 1196. First curve 1195 may be a convex curve having a radius in a range from 2 to 10 mm. In some examples, a radius of first curve 1195 may be 4 mm. In some examples, second curve 1196 may be a concave curve having a radius in a range from 1 to 3 mm. Such tibial components 1004, 1104 may be used in combination with one or more sizes of femoral component 1002.

In a method of using knee joint prosthesis 1000, shown in FIGS. 41-45, femoral component 1002 engages tibial component 1004, and post 1012 is received within central box 1006 of femoral component. In this arrangement, the medial tibial articulation surface and lateral tibial articulation surface engage the medial condylar articulation surface and lateral condylar articulation surface, respectively. Central box 1006 of femoral component 1002 and post 1012 of tibial component 1004 may provide varus-valgus constraint. For example, this restriction of varus-valgus movement may be present over a range of rotational positions from 0 degrees of extension to 110 degrees of flexion. In some examples, this restriction of movement may be present over a range of rotational positions from -10 degrees of extension to 135 degrees of flexion. In still further examples, this restriction of movement may be present over a range of rotational positions from -20 degrees of extension to 150 degrees of flexion. In other examples, the box and the post may be modified to adjust this range.

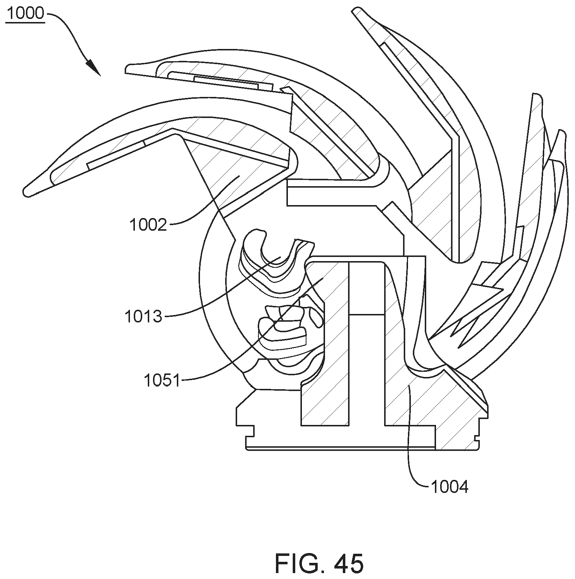

In addition to the varus-valgus constraint, in some examples, femoral cam 1013 and protrusion 1051 of post 1012 of the tibial component 1004 provide anterior-posterior constraint. In some examples, including that depicted in FIGS. 41-45, protrusion 1051 of posterior surface 1045 of post 1012 may engage with femoral cam 1013 of the femoral component 1002. As shown in FIG. 45, this restriction of movement may be present over a range of rotation positions from 0 to 45 degrees flexion (see, e.g., FIGS. 41-42). In such a range of motion, protrusion 1051 on posterior surface 1045 may prevent a femur from moving forward by catching on femoral cam 1013 providing such anterior-posterior stability. As shown in FIGS. 41-42, from 0 to 45 degrees flexion, protrusion 1051 may engage with the femoral cam. Because protrusion 1051 is sloped inferiorly-anteriorly, femoral cam 1013 moves in an anterior direction as flexion increases in this range, preventing the femoral component from sliding posteriorly while also preventing anterior translation by pressing against post 1012. As shown in the sequence of FIGS. 41-44, the femoral cam 1013 remains in contact with the post 1012 throughout the range of motion.

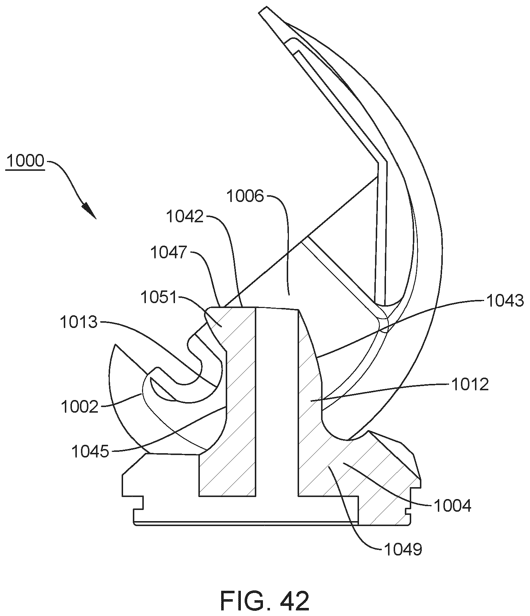

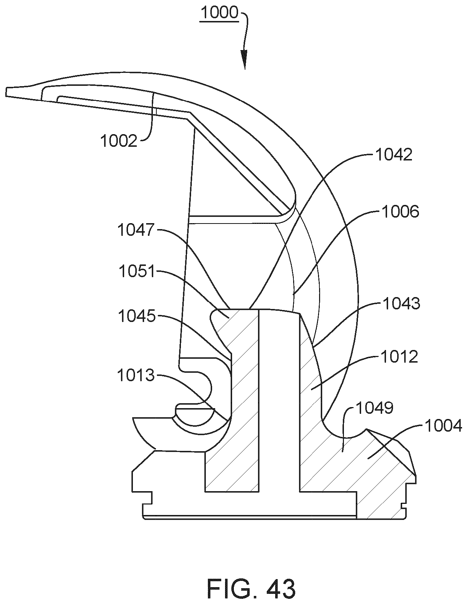

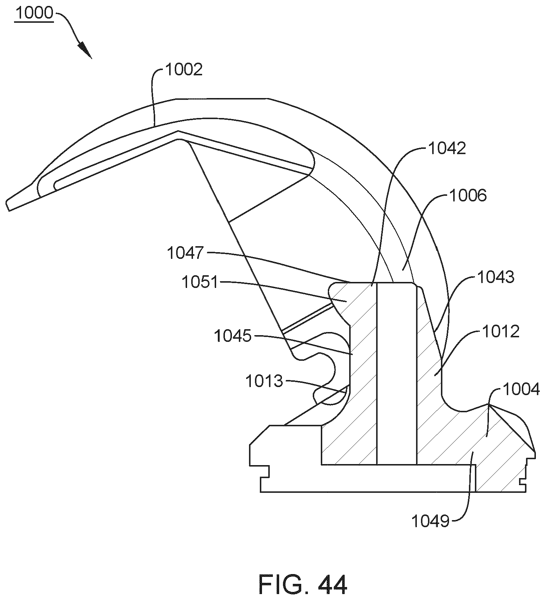

FIG. 41 illustrates knee joint prosthesis 1000 at 10 degrees flexion. FIG. 42 illustrates knee joint prosthesis 1000 at 45 degrees flexion. As shown in FIGS. 43-44, after 45 degrees flexion femoral cam 1013 is below protrusion 1051 and there is no added anterior-posterior constraint. FIG. 43 illustrates knee joint prosthesis 1000 at 90 degrees flexion while FIG. 44 illustrates knee joint prosthesis 1000 at 135 degrees flexion. As shown in FIGS. 43-44 after 45 degrees flexion, femoral cam 1013 engages with posterior surface 1045 of post 1012 to maintain varus-valgus constraint. Tibial component 1104 provides similar constraint to that of tibial component 1004.

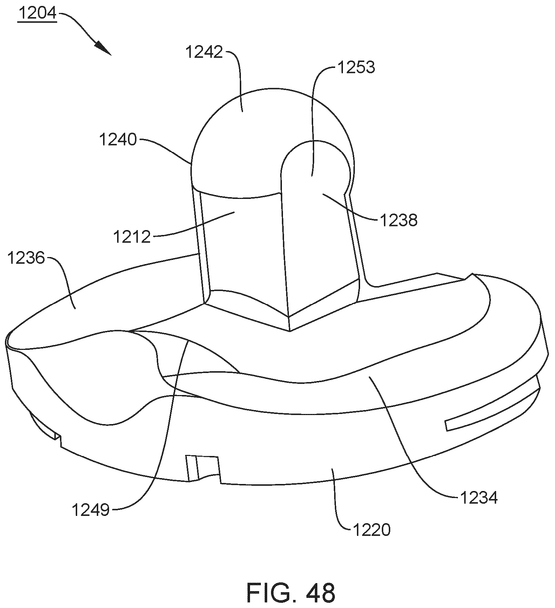

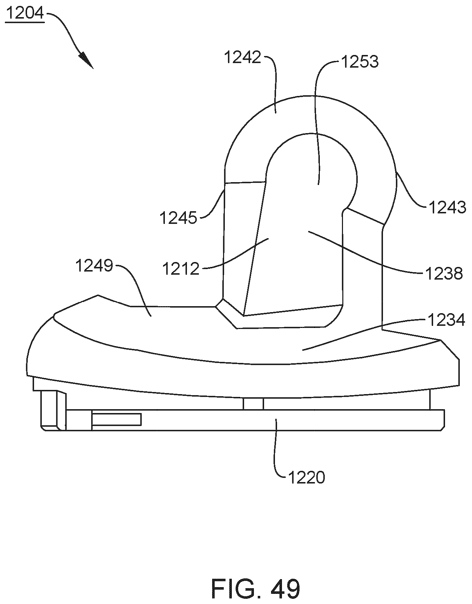

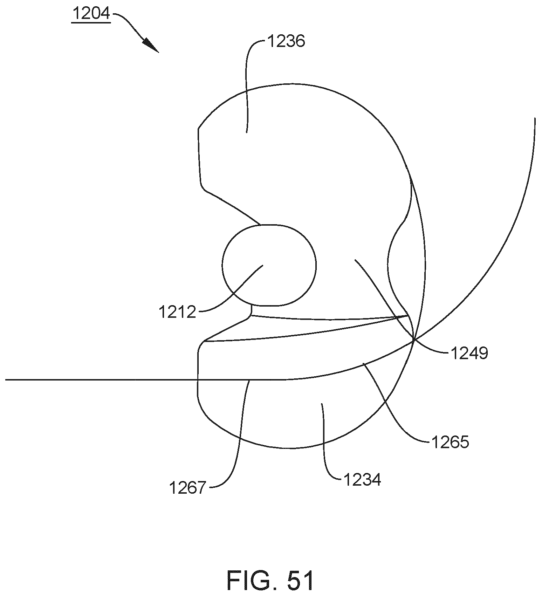

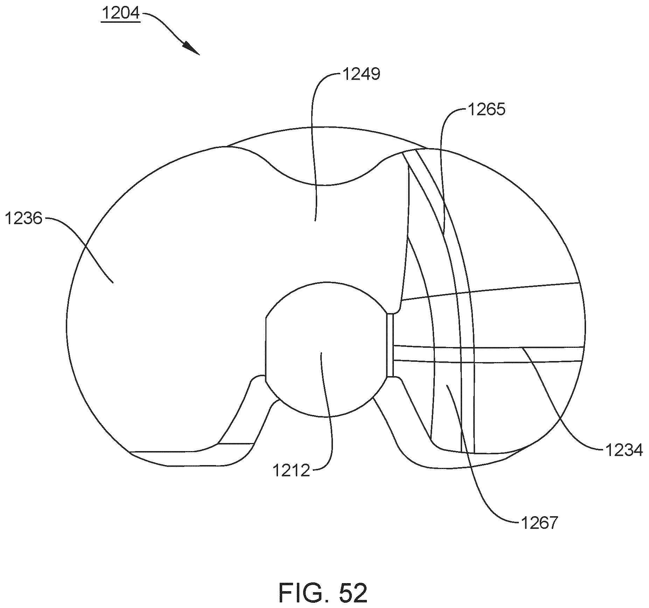

Another embodiment of a tibial component is shown in FIGS. 48-52. For tibial component 1204, like reference numerals refer to like elements of tibial component 104, unless otherwise noted. In some examples, tibial component 1204 may include a medial tibial articulation surface 1234 and a lateral tibial articulation surface 1236, as shown in FIG. 51. Tibial component 1204 further includes a post 1212 positioned between medial and lateral tibial articulation surfaces 1234, 1236. The post 1212 includes a post medial surface 1238, a post lateral surface 1240, and a post central surface 1242 between medial and lateral surfaces of post. Post medial surface 1238 and post lateral surface 1240 each include a respective flat surface portion 1253 extending to the medial and lateral tibial articulation surfaces 1234, 1236, while post central surface 1242 has a convex curved surface portion. In some examples, post central surface 1242 may be hemicylindrical. In some examples, as shown in FIG. 49, a length of a curve of hemicylindrical post central surface 1242 may be longer on an anterior side 1243 of central surface 1242 than a length of the curve on a posterior side 1245 of central surface 1242.

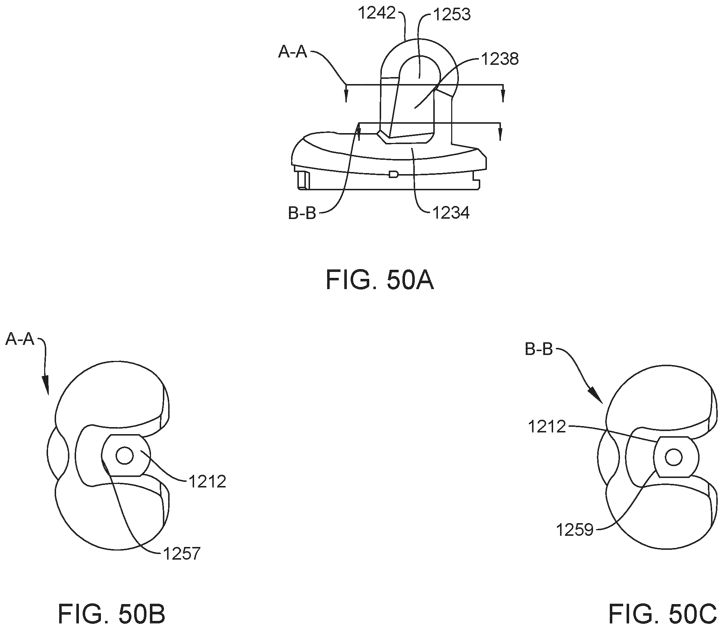

As shown in FIGS. 48-49, flat surface portion 1253 of each of post medial surface 1238 and post lateral surface 1240 may include an angled flat surface having a greater width at a lower portion of flat surface portion than a width at an upper portion of flat surface portion such that the angled flat surfaces define a tapered shape of the post. As shown in FIGS. 50A-50C, dimensions of flat surface portion 1253 are configured such that an anterior radius 1257 of an upper portion of post 1212 (about a central longitudinal axis of the post) is smaller than an anterior radius 1259 of a lower portion of post. As shown in FIG. 50A, flat surface portion 1253 has an increased anterior-posterior width on each of post medial and lateral surfaces 1238, 1240 of post 1212 along line B-B on the lower portion of post relative to a width along line A-A on the upper portion of post. As shown in FIG. 50B-50C, the increased width of flat surface portion 1253 closer to a base of the post is complemented by an increased anterior radius of post 1212. These post characteristics control and limit femoral rotation and anterior-posterior movement.

As shown in FIGS. 51-52, in some examples, tibial component 1204 may include medial tibial articulation surface 1234 and lateral tibial articulation surface 1236 that respectively include an anterior rotary arc 1265 toward an anterior of the tibial component. The anterior rotary arc 1265 may define an inward flare along an anterior portion of the medial and lateral articulation surfaces 1234, 1236. The flared anterior portion of the medial and lateral articulation surfaces 1234, 1236 may facilitate maintaining congruence between corresponding medial and lateral condylar articulation surfaces. In some examples, the anterior rotary arc may accommodate femoral component hyper extension by minimizing distraction of the knee joint prosthesis. As shown in FIGS. 51-52, tibial component 1204 may include medial tibial articulation surface 1234 and lateral tibial articulation surface 1236 that respectively do not include a posterior reverse rotary arc. The anterior rotary arc 1265 may transition into a straight posterior portion 1267. Such an absence of a posterior reverse rotary arc may reduce femoral internal-external rotation during movement of the knee joint through a range of motion.

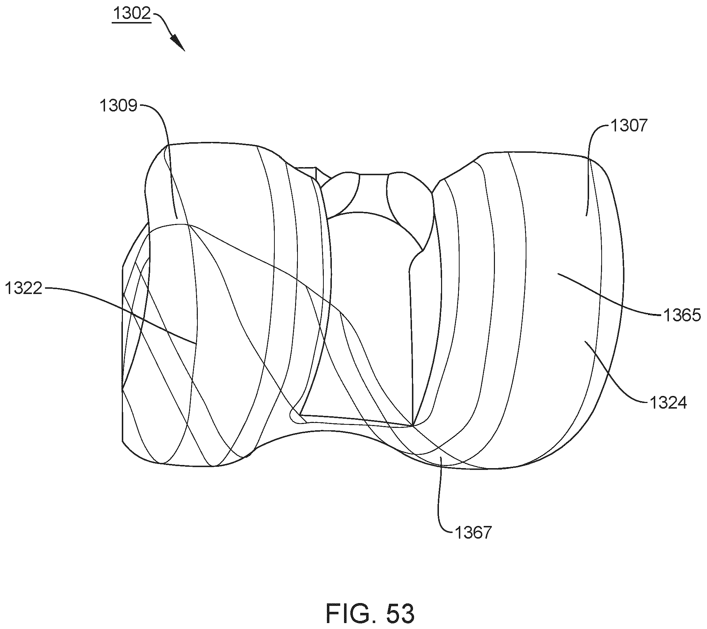

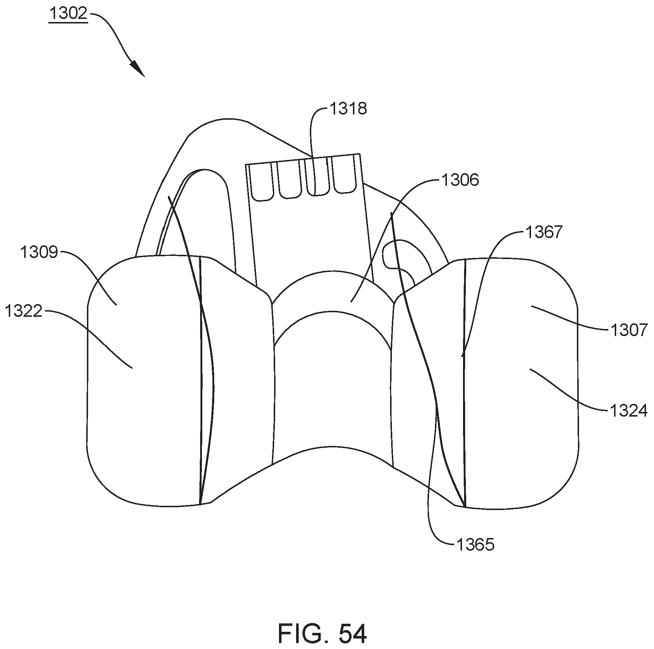

An additional embodiment of a femoral component 1302 is shown in FIGS. 53-54, the design of which is similar to that of femoral component 102, unless otherwise noted. Femoral component 1302 includes a medial condylar articulation surface 1324 and a lateral condylar articulation surface 1322 on medial condylar portion 1307 and lateral condylar portion 1309, respectively. In some examples, medial condylar portion 1307 and lateral condylar portion 1309 may be angled or flared so as to reduce femoral rotation during flexion. In some examples, the medial condylar portion 1307 and lateral condylar portion 1309 may be angled or flared such that medial condylar articulation surface 1324 and a lateral condylar articulation surface 1322 are complimentary to articular surfaces of a tibial component, such as medial tibial articulation surface 1234 and lateral tibial articulation surface 1236 of tibial component 1204 shown in FIGS. 51-52 As such, medial and lateral condylar portions 1307, 1309 each respectively include an anterior flared portion 1365 complimentary to anterior rotary arc 1265 of tibial component 1204 and a straight posterior portion 1367 (straight when viewed in a coronal plane) complimentary to straight posterior portion 1267 of tibial component 1204.

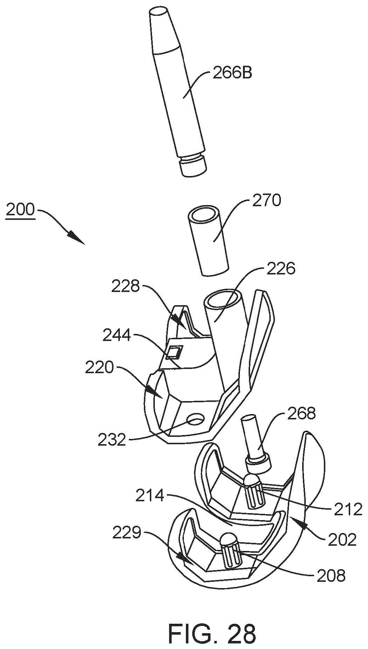

In some examples, the femoral component may further include a proximally extending boss configured to receive a stem to insert the femoral component into the femur bone. In some examples, the proximally extending boss may have an internally tapered bore for mating with a press-fit stem. In other examples, the proximally extending boss may have an internally threaded bore for mating with a threaded cement stem. In some examples, the boss may include an internal tapered bore for mating with a conical press-fit stem. The press-fit stem may include a tapered trunnion type connection with an auxiliary locking screw. In other examples, a threaded cemented stem with a threaded attachment junction can also be used by employing an adapter 270 as shown in FIG. 28. In some examples, the adapter may include a tapered outside surface and a threaded inside surface.

Bone cement may be used in order to secure the femoral component to the femur. In some examples, the bone-facing side of the femoral component may include cement pockets for fixation of the femoral component to the femur. In other examples, the bone-facing side of the femoral component may include ingrowth surfaces for biological fixation of the femoral component to a bone. The ingrowth surfaces may be cemented or press-fitted into the bone.

The knee joint prosthesis as described above may be made of a biocompatible material, such as a polymer e.g., reinforced polymer composites or thermoplastic polymers; a titanium alloy e.g., Ti6Al4V; stainless steel e.g., 316L; or other metals and metal alloys, such as Ti and CoCr. Additionally, the knee joint prosthesis may be manufactured via an additive manufacturing process such as the process described in U.S. Pat. Nos. 4,863,538, 5,017,753, 5,076,869, 4,944,817, 7,537,664, 10,614,176, 11,534,307 and 11,737,880. Each part or component of the knee joint prosthesis may be made from a different material. In particular, in some examples, the post of the tibial component may be made of a polymeric material. In some examples, the tibial inserts may be made of a high-density polyethylene, such as N2VAC or X3. In other examples, the tibial inserts may be made of Titanium 6-4 or CoCr.

In another embodiment, the present disclosure relates to a device for an implant including a femoral component and a tibial component. The femoral component includes a first condylar surface, a second condylar surface, and an enclosure between the first and second condylar surfaces. The enclosure includes a cavity having a second surface within the cavity.

The tibial component includes a first curved surface, a second curved surface, and a peg between each surface. The first and second surfaces are complementary to the first and second condylar surfaces. A first surface of the peg is operatively connected to a second surface within the cavity of the femoral component. The first surface of the peg defines a partially cylindrical end portion of the peg. The peg is curved over the first surface. In other examples, the peg is spherical in shape over the first surface. In some examples, the peg of the tibial component includes a peg medial surface and a peg lateral surface and the cavity of the femoral component includes a medial surface and a lateral surface. The peg medial and lateral surfaces may be angled with respect to the medial and lateral surfaces of the cavity at any angle around or less than 60 degrees.

The femoral component articulates relative to the tibial component over a range of motion. However, medial-lateral movement of the femoral component relative to the tibial component is restrained and anterior-posterior movement of the femoral component relative to the tibial component is restrained. The range of motion with a single axis of rotation may extend from 0 to 110 degrees flexion. A length of the peg is uninterrupted by the femoral component as the peg fits within the cavity of the femoral component, i.e., no part of the femoral component or other attachment component passes into an internal volume of the post. In operation, the first surface of the peg contacts the second surface of the cavity so that as the over the range of motion, the second surface is superior to the first surface.

In some examples, the femoral component includes a bone-facing side having cement pockets for fixation of the femoral component to a bone. In other examples, the femoral component includes a bone-facing side having ingrowth surfaces for biological fixation of the femoral component to a bone. The ingrowth surfaces can be cemented or press-fitted onto the bone. In some examples, the enclosure includes a proximally extending boss having an internally tapered bore for mating with a press-fit stem. In other examples, the enclosure includes a proximally extending boss having an internally threaded bore for mating with a threaded cement stem.

In another aspect, the present disclosure relates to a kit for converting an existing implant into another implant with greater or lesser constraint. For example, an existing implant, such as a cruciate retaining implant, may be converted into a more constrained total stabilizing implant. In another example, a hinge-type implant may be converted into a less constrained total stabilizing implant. In this way, the kit is versatile in that a cruciate retaining or a hinge implant may be converted into a knee joint implant that is more constrained than a total stabilizing implant but less constrained than the hinge implant. The kit allows for easy conversion between implants reducing costs and inventory during surgical knee revisions and replacements. The adaptable implant kit provides significant constraint while preserving natural motion in the knee, unlike a hinge implant, making the implant advantageous where a high degree of constraint is necessary. In some examples, the kit may convert an existing cruciate retaining implant into a posterior stabilizing or total stabilizing implant. Additionally, the kit provides flexibility in having a cementless or a cemented bone fixation option or a conical press-fit stem or a cemented threaded stem.

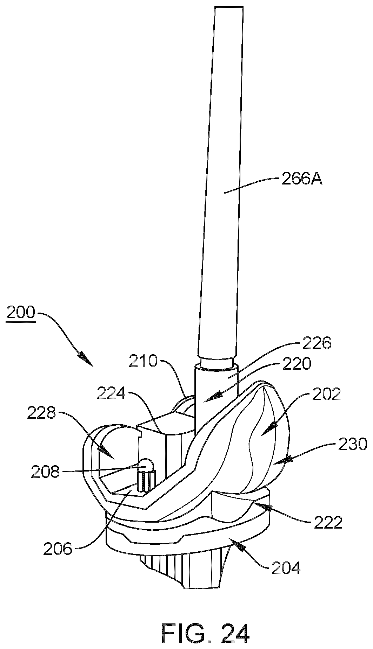

In further detail, with reference to FIGS. 24-28, kit 200 includes an existing implant or a cruciate retaining implant, i.e., a first femoral component 202, a femoral insert 220 configured to be received on a bone-facing side 229 of first femoral component 202, a tibial baseplate 216, a first tibial insert 218 configured to be received on a joint-facing side of the tibial baseplate 216, and a second tibial insert 222. The kit may also include an original tibial insert (not shown). First femoral component 202 includes first and second articulation surfaces and the femoral insert includes a box with a cavity between medial and lateral condylar portions of the femoral insert. When femoral insert 220 is received in first femoral component 202 to define a femoral implant and the first and second tibial inserts 218, 222 are received on first tibial baseplate 216 to define a tibial implant, the femoral implant rotates relative to the tibial implant such that an axis of rotation of the box of the femoral component and the post of the tibial component are coincident over a range of motion. Such interaction between the components is consistent with that described for knee joint prosthesis 100 shown in FIG. 1.

Kit 200 may be configured to convert a first implant, e.g., a cruciate retaining implant into a second implant, which may be an implant with greater constraint or in some cases, a total stabilizing implant. The first implant, in this instance a cruciate retaining implant, includes first femoral component 202 and a first tibial component 204 having first tibial baseplate 216 and an original tibial insert (not shown). First femoral component 202 includes a medial condylar articulation surface 206 that may have a medial peg 208 and a lateral condylar articulation surface 210 that may have a lateral peg 212. First femoral component 202 further includes a central opening 214 between medial and lateral condylar articulation surfaces 206, 210. First tibial component 204 is configured for rotatable attachment to first femoral component 202.

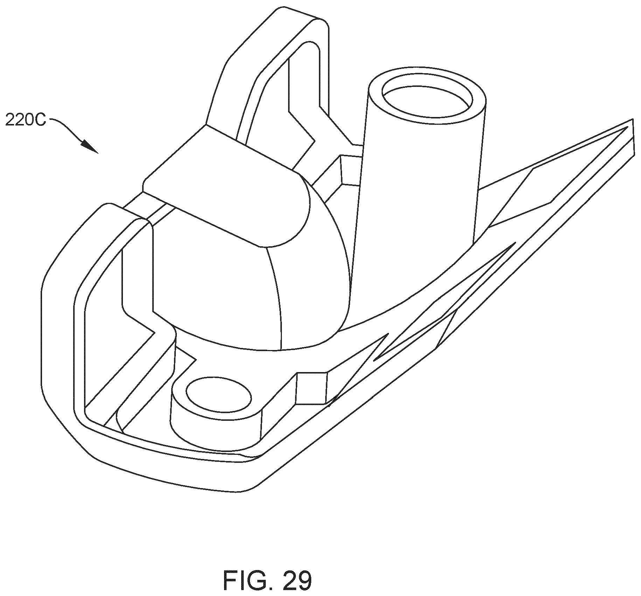

As shown in FIGS. 24-28, femoral insert 220 of kit 200 is receivable on a bone-facing side 229 of first femoral component 202. Femoral insert 220 includes a box 224 with a cavity between medial and lateral condylar portions 206, 210 of the femoral insert 220. Box 224 may have a structure similar to that of central box 106 shown in FIGS. 5-7, unless otherwise noted. Box 224 of femoral insert 220 is enclosed on bone-facing side 229 of femoral component 202 and open on a tibial-facing side 230 of femoral component. A portion of an internal volume of the box 224 is defined by a box medial surface, a box lateral surface, and a box central surface. The box central surface separates the box medial and lateral surfaces. Medial and lateral condylar portions 206, 210 of femoral insert 220 of the second femoral component include a medial opening 232 and a lateral opening 234 complementary to medial and lateral pegs 208, 212 of first femoral component 202. In some examples, bone cement may be configured to secure the first femoral component to the femoral insert to make the second femoral component. The femoral insert is then bone cemented to the first femoral component and attached using the medial and lateral pegs. In a variation of kit 200, the medial and lateral pegs may be modular threaded pegs and may be configured to threadably attach the femoral insert to the first femoral component. In some examples, such fixation may be supplemented by cement fixation. In other examples, the femoral insert is bone cemented to the first femoral component without use of the medial and lateral pegs. In some examples, as shown in FIG. 29, a femoral insert 220C may include openings for bone cement fixation to the first femoral component.

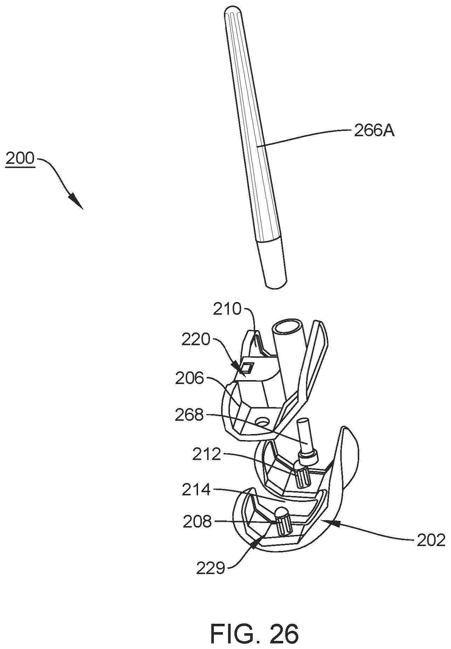

As shown in FIG. 28, kit 200 may further include a stem 266B that is configured to be threaded or press fit into an adapter 270 that is releasably engageable with a boss 226 adjacent to box 224 of femoral insert 220. In some examples, a threaded cemented stem with a threaded attachment junction can also be used by employing an adapter. The adapter may include a tapered outside surface and a threaded inside surface. In other examples, as shown in FIGS. 26-27, kit 200 may include a stem 266A that is configured to be inserted directly into boss 226 of femoral insert 220. In some examples, as shown in FIGS. 26-28, kit 200 may include a fastener 268 that is configured to secure stem 266A, 266B into femoral insert 220. In some examples, fastener 268 may be a bolt, a screw, or a plug.

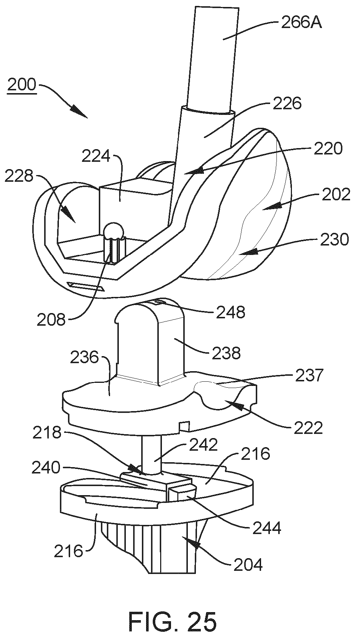

As shown in FIGS. 24 and 25, the tibial components of kit 200 include first tibial component 204 having tibial baseplate 216, a first tibial insert 218, and a second tibial insert 222. In some examples, first tibial insert 218 may include a platform 240 having a peg 242. Platform 240 is positioned between an edge of first tibial component 204 and a protrusion 244 extending transversely from the first tibial baseplate 216. First tibial insert 218 includes peg 242 extending away from first tibial baseplate 216 when first tibial insert is attached to first tibial baseplate. In some examples, second tibial insert 222 may have a bore 248 extending through post 238 configured to receive peg 242 of platform 240. In some examples, first tibial insert 218 is pre-assembled to second tibial insert 222. Pre-assembly may occur at a factory during manufacture of the inserts. The assembly including the first and second tibial inserts 218, 222 may be attached and locked to the first tibial baseplate 216 during surgery.

Second tibial insert 222 of kit 200 includes a post 238 between a medial and a lateral articulation surface 236, 237 of second tibial insert 222. Post 238 may have a structure similar to that of post 112 shown in FIGS. 5-7, unless otherwise noted. Post 238 is receivable within the cavity of box 224 of femoral insert 220. Second tibial insert 222 is configured for rotatable attachment to femoral insert 220 such that the medial-lateral movement of the femoral implant relative to the tibial implant is restrained and the anterior-posterior movement of the femoral implant relative to the tibial implant is restrained. Put another way, when components of the kit are assembled as shown in FIG. 24, the femoral and tibial implants interact in a manner similar to knee joint prosthesis 100.

In one embodiment, a kit may include a femoral insert 220, a first tibial insert 218, and a second tibial insert 222. Such kit may be used to convert an existing cruciate retaining implant into an implant with greater constraint. The femoral insert may attach to a first femoral component of the existing implant and the first and second tibial inserts may attach to a first tibial component of the existing implant. In other examples, a kit may include a plurality of femoral inserts, first tibial inserts, and second tibial inserts configured to convert a plurality of existing cruciate retaining implants into greater constrained implants. In some examples, a kit may include a femoral insert configured to convert an existing femoral component into a femoral component of greater constraint. In yet another example, a kit may include a first tibial insert and second tibial insert configured to convert an existing tibial component into a tibial component of greater constraint. In some of these examples, one or more subsets of inserts in the kit may have a different size than one or more other subsets of inserts in the kit. The above-described kits may also include one or a combination of a stem, a plug, or a bolt (or other fastener(s)), and an adapter for use with an existing stem, and may include any quantity of such components.

In some examples of the kit, the bone-facing side of the femoral insert of the second femoral component may include an ingrowth surface that may secure the second femoral component directly to the femur. An end of the femur bone is cut to have a number of planar surfaces in preparation for engaging the femoral component. In some examples, as shown in FIG. 28, bone-facing side 228 of femoral insert 220 may include around 5 planar surfaces. In other examples, as shown in FIG. 29, bone-facing side 228B of femoral insert 220B may include 3 or 4 planar surfaces depending on the level of bone loss. The more significant the bone loss the lower the number planar surfaces that are available to be cut along an end of the femur bone.

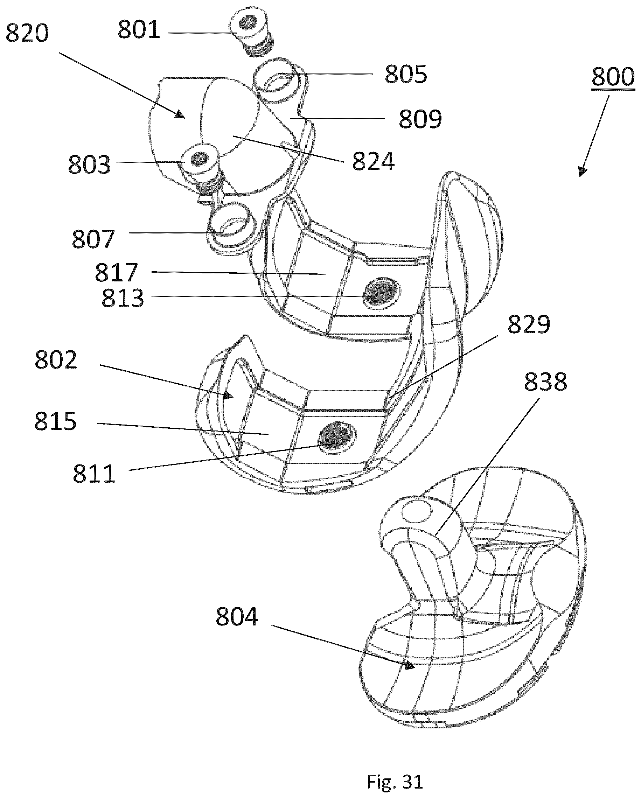

In another embodiment, a kit may be as shown in FIG. 31, and indicated by reference numeral 800. For kit 800, elements in the 800-series of numerals refer to like elements in the 200-series of numerals for kit 200, unless otherwise noted. Kit 800 includes an existing implant, such as a cruciate retaining implant, i.e., a first femoral component 802, a femoral insert 820 configured to be received on a bone-facing side 829 of first femoral component, and a tibial component 804. Kit 800 may be configured to convert a first implant, e.g., a cruciate retaining implant, into a second implant, which may be an implant with greater constraint. The second implant created through the use of kit 800 may be a posterior stabilizing implant or a total stabilizing implant, for example.

We now turn to femoral insert 820 of kit 800. Femoral insert 820 includes a standalone central box 824 with a baseplate 809 extending therefrom in opposite lateral directions, as shown in FIG. 31. Central box 824 may have a structure similar to that of central box 606 as shown in in FIGS. 8-10. In other examples, central box 824 may have a structure similar to that of central box 106 as shown in FIGS. 1-3. In other examples, central box 824 may have a structure similar to that of central box 706 as shown in FIGS. 14-16. Tibial component 804 and post 838 may have a structure similar to that of tibial component 604 and post 612 shown in FIGS. 11-13. In other examples, tibial component 804 and post 838 may have a structure similar to that of tibial component 104 and post 112 shown in FIGS. 5-7. In other examples, tibial component 804 and post 838 may have a structure similar to that of tibial component 704 and post 712 shown in FIGS. 17-19. In other examples, kit 800 may include tibial component 1004 and post 1012 shown in FIG. 46 instead of tibial component 804 and post 838. In other examples, kit 800 may include tibial component 1104 and post 1112 shown in FIG. 47 instead of tibial component 804 and post 838. In other examples, kit 800 may include tibial component 1204 and post 1212 shown in FIGS. 48-50C instead of tibial component 804 and post 838.

Central box 824 may be formed monolithically with baseplate 809 or may be attached to baseplate 809. Baseplate 809 may be used for the fixation of femoral insert 820 to first femoral component 802. Baseplate 809 of femoral insert 820 includes a medial countersink hole 807 and a lateral countersink hole 805 complimentary to a medial screw hole 811 and a lateral screw hole 813 on the medial and lateral condylar articulation surfaces 815, 817 of first femoral component 802. Kit 800 may further include countersink screws 801, 803 to fix femoral insert 820 to first femoral component 802 on either side of central box 824. In some examples, a medial and lateral peg may be used. In other examples, the femoral insert may be fixed to the first femoral component using bone cement or a combination of pegs and bone cement.

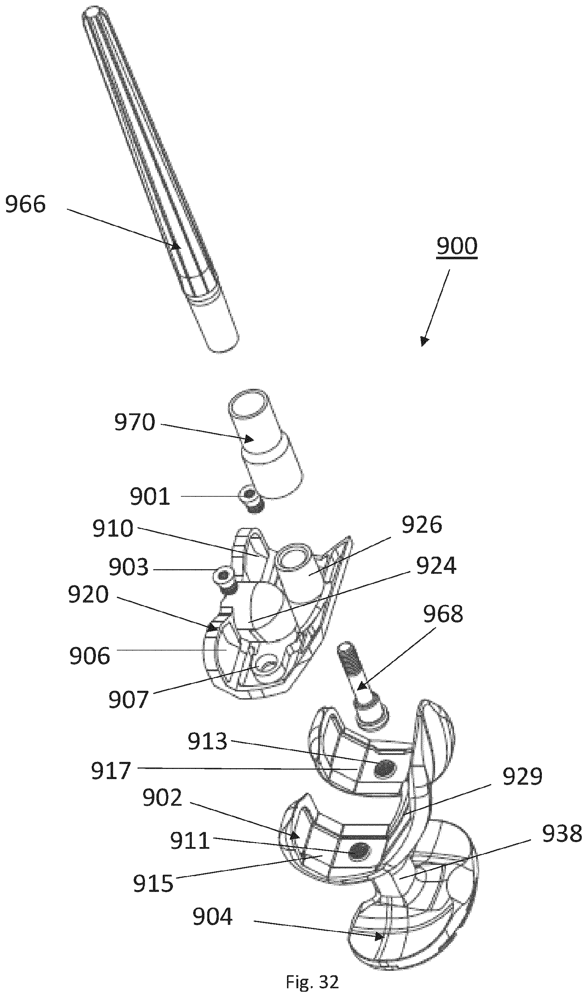

In another embodiment, a kit may be as shown in FIG. 32, and indicated by reference numeral 900. For a kit 900, elements in the 900-series of numerals refer to like elements in the 200-series of numerals for kit 200, unless otherwise noted. Kit 900 includes an existing implant, such as a cruciate retaining implant, i.e., a first femoral component 902, a femoral insert 920 configured to be received on a bone-facing side 929 of first femoral component, and a tibial component 904. Kit 900 may be configured to convert a first implant, e.g., a cruciate retaining implant into a second implant, which may be an implant with greater constraint. The second implant created through use of kit 900 may be a total stabilizing implant or a posterior stabilizing implant, for example.

We now turn to femoral insert 920 of kit 900. Femoral insert 920 includes a standalone central box 924. Central box 924 may have a structure similar to that of central box 706 as shown in in FIGS. 14-16. In other examples, central box 924 may have a structure similar to that of central box 106 as shown in FIGS. 1-3. In other examples, central box 924 may have a structure similar to that of central box 606 as shown in in FIGS. 8-10. Tibial component 904 and post 938 may have a structure similar to that of tibial component 704 and post 712 shown in FIGS. 17-19. In other examples, tibial component 904 and post 938 may have a structure similar to that of tibial component 604 and post 612 shown in FIGS. 11-13. In other examples, tibial component 904 and post 938 may have a structure similar to that of tibial component 104 and post 112 shown in FIGS. 5-7. In other examples, kit 900 may include tibial component 1004 and post 1012 shown in FIG. 46 instead of tibial component 904 and post 938. In other examples, kit 900 may include tibial component 1104 and post 1112 shown in FIG. 47 instead of tibial component 904 and post 938. In other examples, kit 900 may include tibial component 1204 and post 1212 shown in FIGS. 48-50C instead of tibial component 904 and post 938.

Medial and lateral condylar portions 906, 910 of femoral insert 920 include a medial countersink hole 907 and a lateral countersink hole (not shown) on either side of central box 924 complimentary to a medial screw hole 911 and a lateral screw hole 913 on medial and lateral condylar articulation surfaces 915, 917 of first femoral component 902. Kit 900 may further include countersink screws 901, 903 to fix femoral insert 920 to first femoral component 902 on either side of central box 924. In some examples, a medial and lateral peg may be used. In other examples, the femoral insert may be fixed to the first femoral component using bone cement or a combination of pegs and bone cement. In some examples, kit 900 may include a fastener 968 that is configured to secure stem 966 and into femoral insert 920. In some examples, fastener 968 may be a bolt, a screw, or a plug. Stem 966 may be threaded or press fit into an adapter 970 that is releasably engageable with a boss 926 adjacent to central box 924 of femoral insert 920. In some examples, adapter 970 is tapered to accept stem 966. In some examples, boss 926 may be separately attached to femoral insert 920. In other examples, boss 926 may directly from femoral insert 920. In some examples, boss 926 may be tapered to accept femoral insert 920.

In some examples, such a kit may be used to convert an existing cruciate retaining implant into an implant providing greater constraint. The femoral insert may attach to a first femoral component of the existing implant. In further examples, a kit may include a first tibial insert configured to convert an existing tibial component into a tibial component of greater constraint. In other examples, a kit may include a first tibial insert and a second tibial insert to convert an existing tibial component into a tibial component of greater constraint. In some of these examples, one or more subsets of inserts in the kit may have a different size than one or more other subsets of inserts in the kit. The above-described kits may also include one or a combination of a stem, a fastener, and an adapter for use with an existing stem, and may include any quantity of such components.

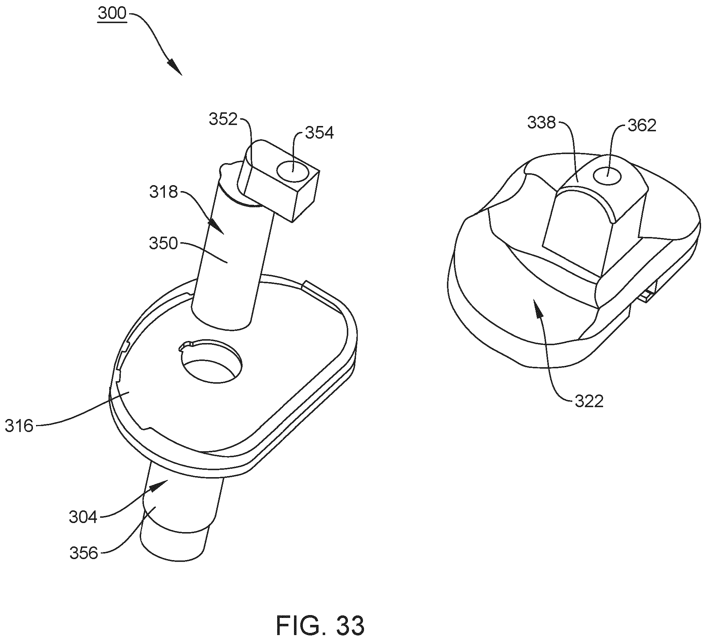

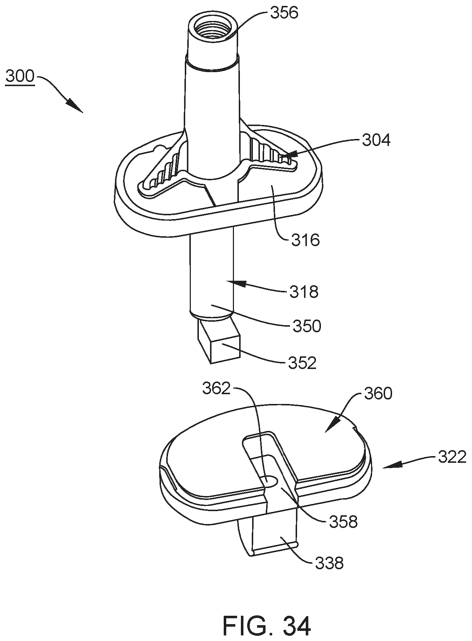

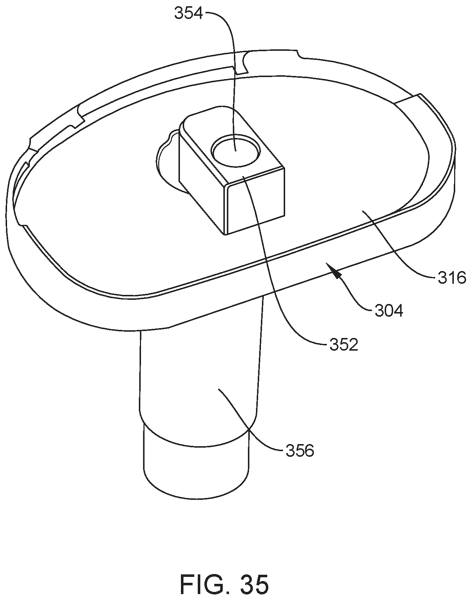

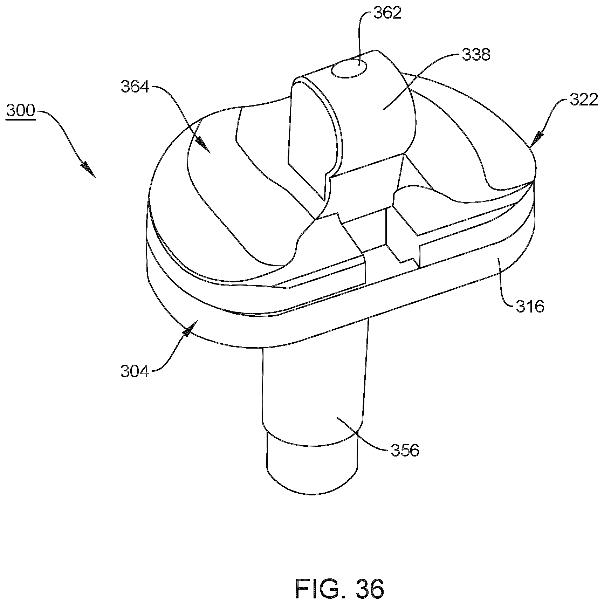

In yet another embodiment shown in FIGS. 33-38, a kit 300 may include a femoral component as described with reference to kit 200. However, kit 300 is configured to convert a tibial component of a hinge implant into an implant more constrained than a total stabilizing tibial component and less constrained than the hinge implant. Kit 300 includes a first tibial component 304 including a boss 356, a first tibial insert 318 having an elongated peg 350, and a second tibial insert 322 having a post 338. Post 338 may have a structure similar to that of post 112 shown in FIGS. 5-7, unless otherwise noted. First tibial insert 318 and second tibial insert 322 are configured to attach to first tibial component 304 to form the second tibial component of lesser constraint. A first tibial insert 318 may include an elongated peg 350 having a peg 352 including a bore 354. Elongated peg 350 is configured to be inserted into boss 356 extending from first tibial baseplate 316 of first tibial component 304 so that end of elongated peg protrudes from first tibial baseplate after elongated peg is fully inserted into boss. In some examples, boss 356 may have a threaded interior bore which is complementary to a threaded portion of elongated peg 350. A second tibial insert 322 is configured to engage peg 352 of elongated peg 350. Second tibial insert 322 may include a central opening 358 proximate to a post 338 on a tibial-facing side 360 of second tibial insert and is configured to receive peg 352 of elongated peg 350. Post 338 may include a bore 362 extending through post to central opening 358, bore extending from femur-facing side 364 to tibial-facing side 360.

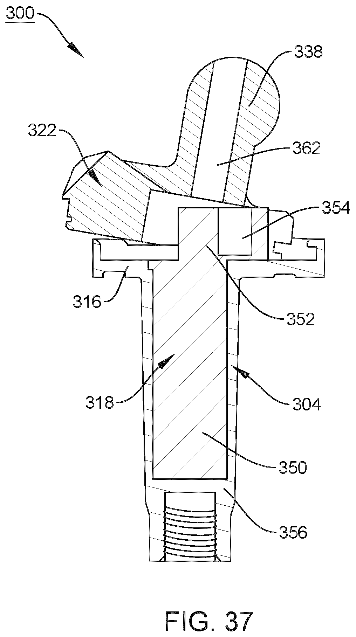

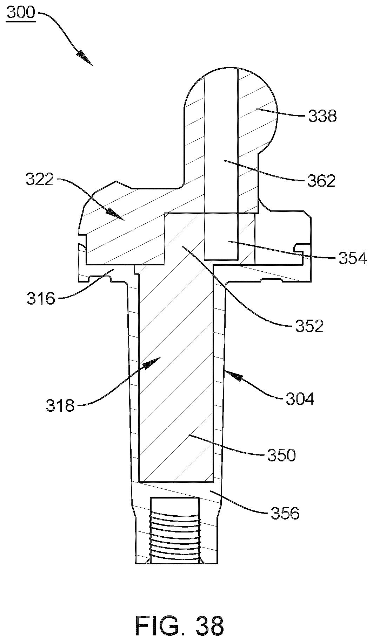

With continued reference to kit 300, as shown in FIGS. 37 and 38, in some examples second tibial insert 322 is configured so that it may be placed on first tibial insert 318 by angling second tibial insert posteriorly onto first tibial insert. Second tibial insert 322 is pushed until it snaps into place anteriorly. Bore 354 of peg 352 of elongated peg 350 and bore 362 of post 338 are aligned when the second tibial insert 322 is correctly seated on first tibial insert 318. Then, a fastener or stabilizer pin may be inserted into the bore of the post to fix the second tibial insert to the first tibial insert.

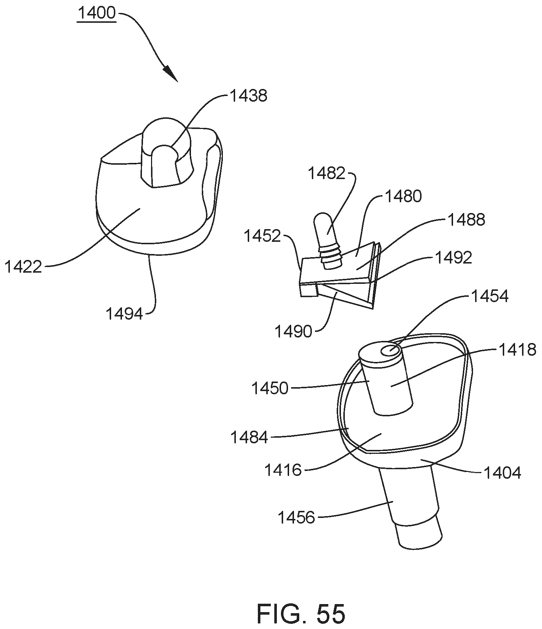

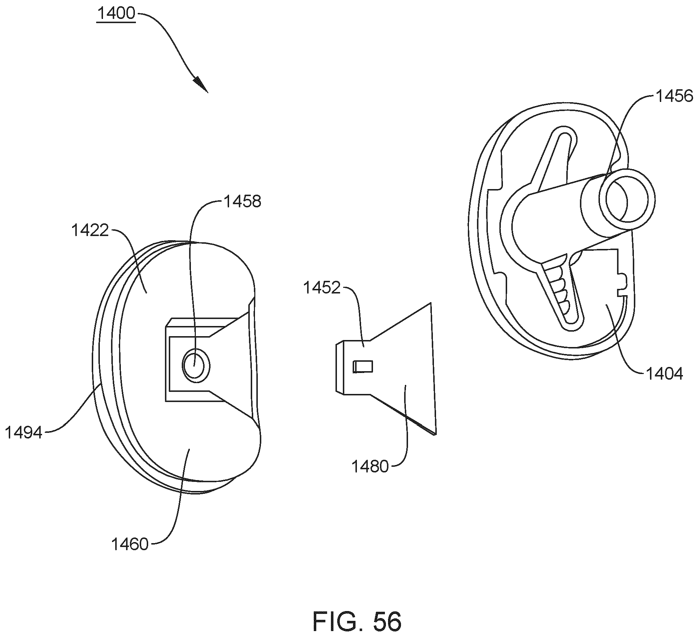

In yet another embodiment shown in FIGS. 55-59, a kit 1400 may include a femoral component as described with reference to femoral component 102. Kit 1400 may be configured to convert a tibial component of a total stabilizing implant into a hinge implant. Kit 1400 may include a first tibial component 1404 including a boss 1456, a first tibial insert 1418 having an elongated peg 1450, and a second tibial insert 1452. Kit 1400 may further include a third tibial insert 1422 having a post 1438. Post 1438 may have a structure similar to that of post 1212 shown in FIGS. 48-50C, unless otherwise noted. First, second, and third tibial inserts 1418, 1452, 1422 are configured to attach to first tibial component 1404. In some embodiments of kit 1400, the kit may include tibial components without femoral components.

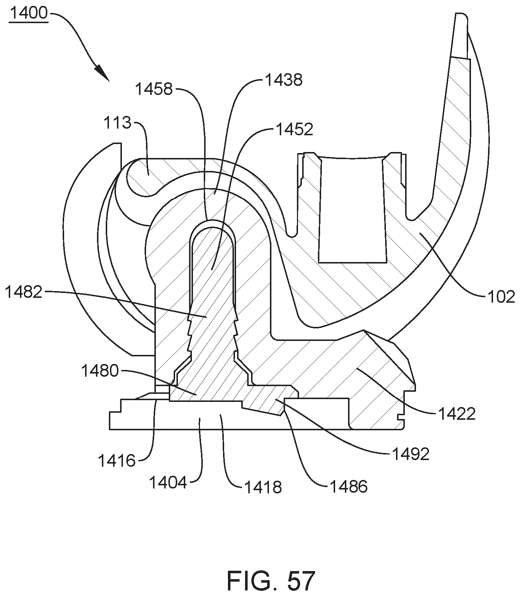

First tibial component 1404 includes a first tibial baseplate 1416. First tibial baseplate 1416 may include an edge 1484 around the periphery of first tibial baseplate. Edge 1484 may include one or more recesses. In some examples, first tibial baseplate includes a boss 1456. First tibial insert 1418 may include an elongated peg 1450 including a bore 1454. Elongated peg 1450 is configured to be inserted into boss 1456 extending from first tibial baseplate 1416 of first tibial component 1404. In some examples, boss 1456 may have a threaded interior bore which is complementary to a threaded portion of elongated peg 1450. As shown in FIG. 57, first tibial baseplate 1416 may include a recess 1486 adjacent boss 1456 (shown in FIG. 55). First tibial insert 1418 may attach to second tibial insert 1452. In some examples, second tibial insert 1452 may be adhered to an upper surface of elongated peg 1450. In other examples, second tibial insert 1452 may be fixed to elongated peg 1450 using a fastener which is configured to extend through bore 1454 (FIG. 55). In some examples, second tibial insert 1452 may attach to both an end of elongated peg 1450 and first tibial baseplate 1416.

In some examples, second tibial insert 1452 may include a base portion 1480 and a peg portion 1482. Base portion 1480 may be a flat surface having a generally triangular or trapezoidal shape. Base portion 1480 may include a rectangular platform 1488 extending upward from a triangular portion 1490. Triangular portion 1490 of base portion 1480 may include a step portion 1492 forming a ridge. As shown in FIG. 57, the ridge of base portion 1480 may be received within recess 1486 of first tibial baseplate 1416. Base portion 1480 may be attached to peg portion 1482. Peg portion 1482 may protrude upward from a center point of rectangular platform 1488 of base portion 1480. In some examples, second tibial insert 1452 may be monolithic. In some examples, peg portion 1482 may have one or more ridges extending across a circumference of peg portion. Peg portion 1482 may have a cylindrical body.

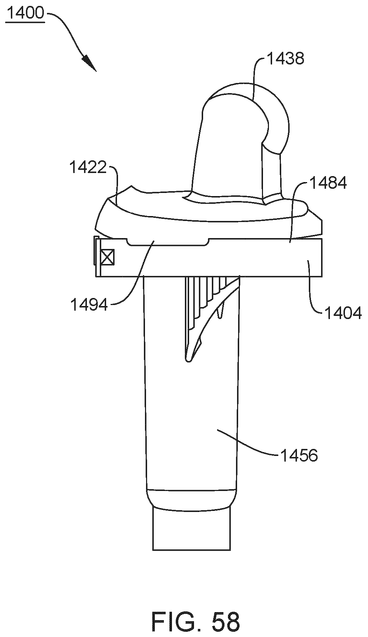

In some examples, as shown in FIGS. 55-56, third tibial insert 1422 may include a central opening 1458 within a post 1438 on a tibial-facing side 1460 of third tibial insert. Central opening 1458 may be configured to receive peg portion 1482 of third tibial insert 1422. Third tibial insert 1422 is configured to engage second tibial insert 1452. Central opening 1458 may have one or more grooves configured to receive the one or more ridges of peg portion 1482. In some examples, second tibial insert 1452 may be made from a metal and third tibial insert 1422 may be made from a polyethylene. In such instances, the one or more ridges of the peg portion 1482 may be configured to prevent backout of second tibial insert 1452 from central opening 1458 of third tibial insert 1422. As shown in FIG. 57, peg portion 1482 of second tibial insert 1452 may be received within central opening 1458 of third tibial insert 1422. In some examples, as shown in FIG. 58, third tibial insert 1422 may include one or more grooves along a periphery of an edge 1494 of third tibial insert complimentary to edge 1484 of first tibial component 1404. The one or more grooves of third tibial insert 1422 are configured to be received within the recesses of edge 1484 of first tibial component 1404 to secure third tibial insert to first tibial component.

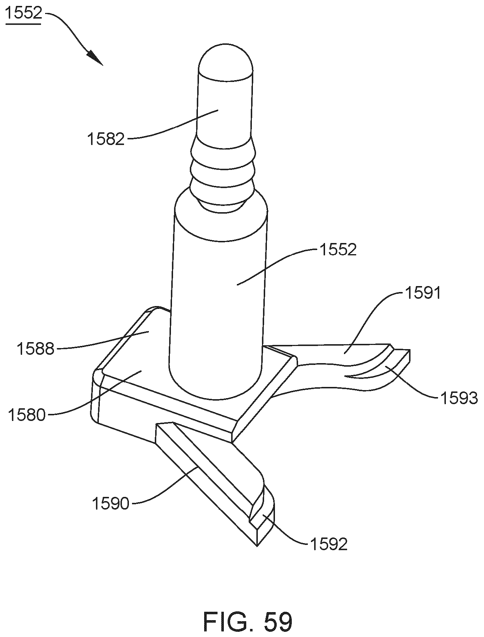

In further examples, as shown in FIG. 59, second tibial insert 1552 may have a design similar to that of second tibial insert 1452, unless otherwise noted. However, as shown in FIG. 59, second tibial insert 1552 may include base portion 1580 having a rectangular platform 1588 connecting to a first 1590 and second arm 1591. First and second arms 1590, 1591 may extend from opposing ends of rectangular platform 1588. First and second arms 1590, 1591 of base portion 1580 may each include respective step portions 1592, 1593 forming a ridge. Peg portion 1582 may extend from rectangular platform 1588 of base portion 1580. Peg portion 1582 may have a first portion and a second portion. The first portion may have a radius greater than a radius of the second portion. The second portion may have one or more ridges extending across a circumference of the peg portion 1582. Peg portion 1582 may be cylindrical in shape. Second tibial insert 1552 may be used in combination with kit 1400 as described above and assembled with first tibial insert 1418, third tibial insert 1422, and first tibial component 1404.

In some embodiments, a kit may include a femoral insert, a first tibial insert, and a second tibial insert. Such kit may be used to convert an existing hinge implant into a lesser constraint implant. The femoral insert may attach to a first femoral component of the existing implant and the first and second tibial inserts may attach to a first tibial component of the existing implant. In other examples, a kit may include a plurality of femoral inserts, first tibial inserts, and second tibial inserts configured to convert a plurality of existing hinge implants into lesser constraint implants. In further examples, a kit may include a first tibial insert and second tibial insert configured to convert an existing tibial component into a tibial component of lesser constraint. In some of these examples, one or more subsets of inserts in the kit may have a different size than one or more other subsets of inserts in the kit. The above-described kits may also include one or a combination of a stem, a fastener, and an adapter for use with an existing stem, and may include any quantity of such components.

In still further embodiments, a kit may include a femoral insert and one or more sets of first and second tibial inserts. In any one of the contemplated embodiments, the components of a kit may be provided in a single package. In other variations, each component may be provided in an individual package or the kit may include a subset of components in a single package with the remaining components being individually packaged.

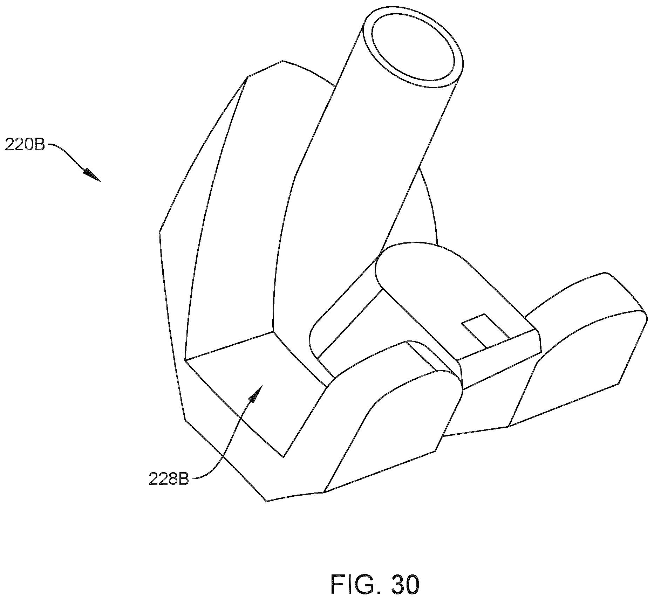

In another aspect, the present disclosure relates to a system inclusive of the assembled components of a kit 200, 300, 800, 900, and 1400. For example, one embodiment of a system may include each component of kit 200 shown in FIGS. 24-28 in an assembled condition so that the implant system functions as a total stabilizing knee implant. In another embodiment, a system may include each component of kit 300 shown in FIGS. 33-38 in an assembled condition along with one of the femoral components shown in FIGS. 26-30, also in an assembled condition, so that the implant system functions as a total stabilizing knee implant. In a further embodiment, a system may include each component of kit 800 shown in FIG. 31 in an assembled condition so that the implant system functions as posterior stabilizing knee implant. In yet another embodiment, a system may include each component of kit 900 shown in FIG. 32 in an assembled condition so that the implant system functions as total stabilizing knee implant. In yet another embodiment, a system may include each component of kit 1400 shown in FIG. 55 in an assembled condition so that the implant system functions as total stabilizing knee implant.

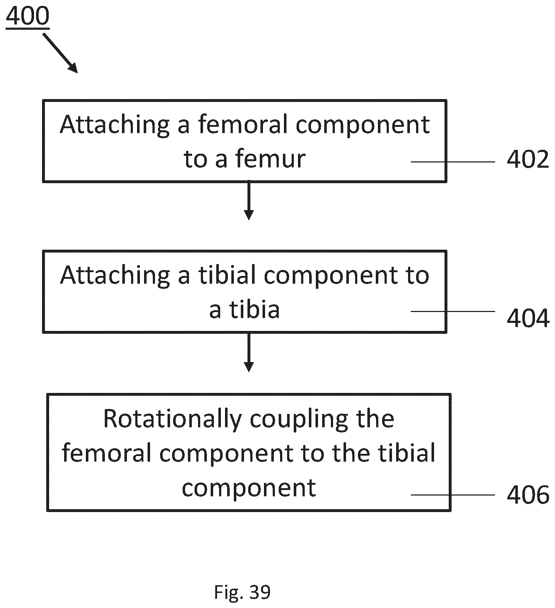

In another aspect, the present disclosure relates to a method for implanting a knee joint prosthesis. One embodiment of such method is method 400 shown in FIG. 39. Method 400 of implanting a knee joint prosthesis includes a step 402 of attaching a femoral component to a femur, a step 404 of attaching a tibial component to a tibia, and a step 406 of rotationally coupling the femoral component to the tibial component.

In some embodiments, a method 400 of implanting a knee joint prosthesis is performed with knee joint prosthesis 100 shown in FIG. 1. In this method 400, step 402 involves attaching a femoral component 102 to a femur. In some examples, attaching femoral component 102 to the femur may include attaching a stem to the femoral component. The stem may be attached to the femur before or with the femoral component. In some examples, attaching the stem to femoral component 102 includes applying a bone cement on an interface between stem and femoral component. In other examples, attaching the stem to femoral component 102 includes press-fitting stem into femoral component. The stem may be threaded and inserted into a threaded bore of a boss 118 of femoral component 102. The stem may be a fluted stem or a cemented stem, and a stem extender may be used. In some examples, femoral component 102 may include a bone-facing surface 108 that has cement pockets to attach femoral component to the femur. In some examples, bone-facing surface 108 may include ingrowth surfaces that bond with the bone surface of the femur to secure femoral component 102.

The femur is prepared by a distal femoral resection in which the planar surfaces of the bone are prepared to be complementary to the planar surfaces of the femoral component. After the planar surfaces are resected along the end of the femur, a pilot hole is drilled into the bone surface in preparation for inserting the stem. A femoral bone cone augment may optionally be used such as a central femoral cone for added stability. Once, the femoral bone is prepared, the stem is inserted into the femur. The stem may be directly secured to the boss. In other examples, an adapter may be used for added flexibility to account for an offset between the pilot hole drilled in the femur and the stem size necessary to fix within the bore of the boss. The adapter is inserted within the bore of the boss in the femoral component and the stem is fixed to a bore within the adapter. The stem may be secured within the adapter via a threaded portion or a press fit mechanism.

Step 404 of attaching a tibial component 104 to the tibia includes using bone cement or an ingrowth surface similar to that of femoral component 102. Tibial component 104 may include a fluted keel or a cemented keel. In some examples, an interior portion of the tibia will be removed to allow the tibial component to fully be seated on the bone. After the tibia is shaved or cut to a flat surface, a pilot hole is drilled into the bone surface. In some examples, a tibial cone augment may be used for added stability. The cone may be symmetric or asymmetric depending on the size and geometry of the bone defect. Once the tibial bone surface is prepared to receive the tibial component, the tibial component is positioned onto the bone surface for securement thereto.

Step 406 of rotationally coupling femoral component 102 to tibial component 104 involves engaging tibial component with femoral component. Post 112 of tibial component 104 is inserted into central box 106 of femoral component 102. In particular, post medial surface 138 is complementary to box medial surface 105, post lateral surface 140 is complementary to box lateral surface 103, and post central surface 142 is complementary to box central surface 101. Rotationally coupling femoral component 102 to tibial component 104 includes inserting post 112 of tibial component into central box 106 of femoral component so that femoral and tibial component can move relative to each other about an axis of rotation 116.

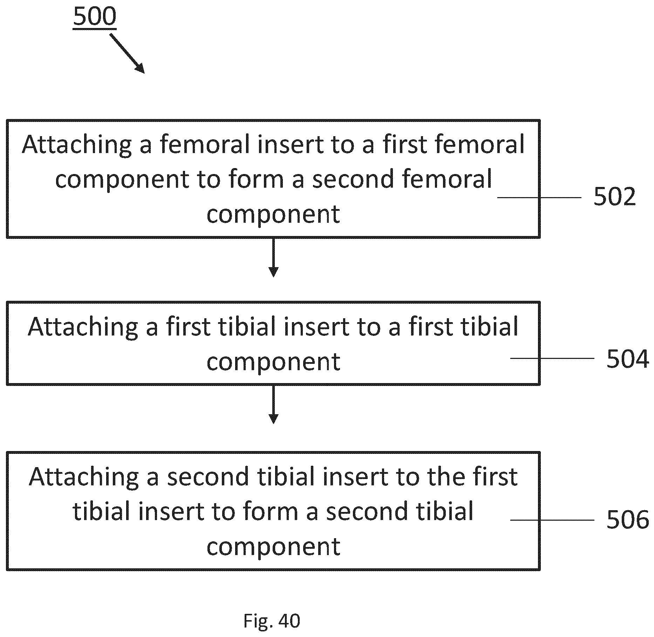

In another aspect, the present disclosure relates to a method of assembly for converting an existing knee joint prosthesis. One embodiment of such method is method 500 shown in FIG. 40. Method 500 of converting an existing knee joint prosthesis includes a step 502 of attaching a femoral insert to a first femoral component to form a second femoral component, a step 504 of attaching a first tibial insert to a first tibial component, and a step 506 of attaching a second tibial insert to the first tibial insert to form the second tibial component.

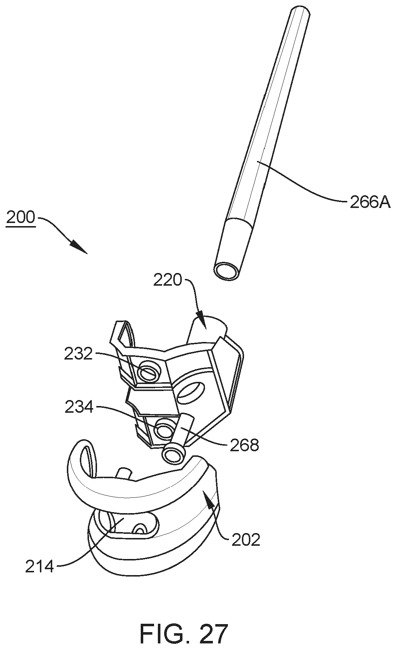

In some embodiments, a method 500 of converting an existing knee joint prosthesis is shown in FIGS. 24-28. In this method 500, step 502 includes attaching a femoral insert 220 to first femoral component 202. As shown in FIGS. 27-28, femoral insert 220 is attached to a bone-facing side 229 of first femoral component 202. Femoral insert 220 includes medial and lateral openings 232, 234 which receive complimentary medial and lateral pegs 208, 212 of first femoral component 202 when femoral insert 220 is attached to first femoral component 202. Medial and lateral pegs 208, 212 are inserted through medial and lateral openings, securing femoral insert 220 to first femoral component 202 to form the second femoral component. Optionally, fastener 268 may be received through femoral insert 220, as shown in FIG. 27, to aid in the attachment of stem 266A prior to attachment of femoral insert 220 to first femoral component 202. An opening in femoral insert 220 is sized so that use of fastener 268 does not interfere with the articular surfaces of first femoral component 202. Step 504 includes attaching a first tibial insert 218 to a first tibial component 204 as shown in FIGS. 24-25. First tibial insert 218 is secured to a first tibial baseplate 216 of first tibial component 204. First tibial insert 218 includes a peg 242. The first tibial insert may be secured to the first tibial baseplate of the first tibial component using a friction fit or a latch mechanism between the baseplate and insert, or other established engagement mechanisms.

Step 506 includes attaching a second tibial insert 222 to first tibial insert 218 to form the second tibial component as shown in FIGS. 24-25. Second tibial insert 222 is attached to first tibial insert 218 and first tibial baseplate 216. Second tibial insert 222 is secured to first tibial baseplate 216 by being inserted over peg 242 of first tibial insert 218. Peg 242 of first tibial insert is complimentary to a bore 248 extending through a post 238 of second tibial insert 222. Second tibial insert 222 may be attached to first tibial baseplate 216 using a press fit mechanism. The second tibial insert may be secured to the first tibial baseplate using a press fit mechanism. In some examples, first tibial insert 218 and second tibial insert 222 are pre-assembled. Such pre-assembly may be in a factory separate from a surgical site.

In other embodiments, a method of assembly may involve components of kit 300 as shown in FIGS. 33-38 to convert a hinge knee implant to a total stabilizing implant. The steps of such method of assembly may be the same as those described for the components of kit 200, unless otherwise noted. In some embodiments of the method of assembly using components of kit 300, a step 504 of attaching first tibial insert 318 to first tibial component 304 may include first attaching first tibial insert 318 having an elongated peg 350 to first tibial component having a first tibial baseplate 316 and a boss 356 as shown in FIGS. 33-38. Boss 356 of first tibial component 304 may have a hollow interior configured to receive elongated peg 350 of first tibial insert 318. Elongated peg 350 is inserted within boss 356 of first tibial component 304. Elongated peg 350 of first tibial insert 318 includes a peg 352 that protrudes from first tibial baseplate 316 after elongated peg is fully inserted into boss 356.

In some embodiments, a step 506 of attaching second tibial insert 322 to the first tibial insert 318 may include securing a central opening 358 of second tibial insert with peg 352 of elongated peg 350 of first tibial insert 318 as shown in FIGS. 33-38. Second tibial insert 322 may be secured to first tibial insert 318 and first tibial baseplate 316 using a press fit mechanism, bone cement, or a combination of both. Peg 352 of first tibial insert 318 includes a bore 354 that aligns with a bore 362 extending through a post 338 of second tibial insert 322 after second tibial insert is attached to first tibial insert. After second tibial insert 322 is placed over first tibial insert 318, a fastener or stabilizer pin may be inserted into aligned bores 354, 362 extending through first tibial insert and second tibial insert to secure them together.

It is to be understood that the disclosure set forth herein includes any possible combinations of the particular features set forth above, whether specifically disclosed herein or not. For example, where a particular feature is disclosed in the context of a particular aspect, arrangement, configuration, or arrangement, that feature may also be used, to the extent possible, in combination with and/or in the context of other particular aspects, arrangements, configurations, and arrangements of the technology, and in the technology generally.

Although the disclosure herein has been described with reference to particular embodiments, it is to be understood that these embodiments are merely illustrative of the principles and applications of the present disclosure. For example, features described in relation to one particular embodiment may be combined with features of other embodiments described herein. It is therefore to be understood that numerous modifications may be made to the illustrative embodiments and that other arrangements may be devised without departing from the scope of the present disclosure as defined in the appended claims.

## Claims

1. An implant (100, 600, 700, 1000), comprising:
a femoral component (102, 602, 702, 1002) including a first condylar surface (107, 607, 707), a second condylar surface (109, 609, 709), and a box (106, 606, 706, 1006) positioned between the first and the second condylar surfaces (107, 109, 607, 609, 707, 709), the box (106, 606, 706, 1006) including a cavity; and
a tibial component (104, 604, 704, 1004, 1104) including a first curved surface (134, 634, 734), a second curved surface (136, 636, 736), and a post (112, 612, 712, 1012, 1112) between the first and second curved surfaces, the first and second curved surfaces being complementary to the first and the second condylar surfaces (107, 109, 607, 609, 707, 709), respectively,
wherein a first surface (142, 642, 742, 1042, 1142) on the post (112, 612, 712, 1012, 1112) of the tibial component (104, 604, 704, 1004, 1104) is operatively connected to a second surface (101, 601, 701) within the cavity of the femoral component (102, 602, 702, 1002), and
wherein when the femoral component (102, 602, 702, 1002) articulates relative to the tibial component (104, 604, 704, 1004, 1104) a medial-lateral movement of the femoral component relative to the tibial component is restrained over a first range of motion and an anterior-posterior movement of the femoral component relative to the tibial component is restrained over a second range of motion.

2. The implant of claim 1, wherein the second surface (101, 601, 701) within the cavity of the femoral component (102, 602, 702) is a concave curved surface portion in an anterior-posterior direction, and the first surface (142, 642, 742) of the post is a convex curved surface portion and wherein the second surface travels along the first surface of over the first range of motion such that an axis of rotation of the second surface of the femoral component (102, 602, 702) and the first surface of the tibial component (104, 604, 704) is coincident over the first and second range of motion.

3. The implant of claim 1 or claim 2, wherein the femoral component (1002) articulates relative to the tibial component (1004, 1104) over the first range of motion and an anterior-posterior movement of the femoral component relative to the tibial component is restrained over the second range of motion and wherein the second range of motion ranges from 0 degrees flexion to 45 degrees flexion.

4. The implant of claim 3, wherein the first surface on the post of the tibial component (1004, 1104) includes a protrusion (1051, 1151) that is operatively connected to the second surface within the cavity of the femoral component (1002) over the second range of motion.

5. The implant of any one of claims 1-4, wherein the first surface (142, 642, 742) defines a partially cylindrical end portion of the post (112, 612, 712).

6. The implant of any one of claims 1-5, wherein the first range of motion and the second range of motion range from 0 degrees flexion to 110 degrees flexion.

7. The implant of any one of claims 1-6, wherein a length of the post (112, 612, 712) is uninterrupted by the femoral component (102, 602, 702).

8. The implant of any one of claims 1-7, wherein over the first range of motion and the second range of motion, the second surface (101, 601, 701) is superior to the first surface (142, 642, 742).

9. The implant of any one of claims 1-8, wherein the femoral component (102) includes a bone-facing side (108) having cement pockets for fixation of the femoral component to a bone.

10. The implant of any one of claims 1-9, wherein the femoral component (102) includes a bone-facing side (108) having ingrowth surfaces for biological fixation of the femoral component to a bone.

11. The implant of any one of claims 1-10, further comprising a proximally extending boss (118) having an internally tapered bore for mating with a press-fit stem (266A).

12. The implant of any one of claims 1-11, further comprising a proximally extending boss (118) having an internally threaded bore for mating with a threaded cement stem (266A).

13. The implant of any one of claims 1-12, wherein the post (612) of the tibial component (604) includes a post medial surface (638) and a post lateral surface (640) and the cavity of the femoral component (602) includes a medial surface (605) and a lateral surface (603), the post medial and lateral surfaces being angled with respect to the respective medial and lateral surfaces of the cavity by an angle of less than 60 degrees.
